(19)

Europäisches
Patentamt
European
Patent Office
Office européen
des brevets

(11) **EP 3 251 737 A1**

(12) **EUROPEAN PATENT APPLICATION**

(43) Date of publication:
**06.12.2017 Bulletin 2017/49**

(21) Application number: **16172170.9**

(22) Date of filing: **31.05.2016**

(51) Int Cl.:
*B01D 61/02* (2006.01)  *C07C 37/82* (2006.01)
*C07C 37/88* (2006.01)  *C07C 39/06* (2006.01)
*C07C 39/08* (2006.01)  *C07C 39/19* (2006.01)
*C07C 51/47* (2006.01)  *C07C 51/50* (2006.01)
*C07C 65/05* (2006.01)  *C07C 65/19* (2006.01)
*C07C 27/26* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Evonik Degussa GmbH**
**45128 Essen (DE)**

(72) Inventors:
• **HEYMANN, Jennifer**
 **40477 Düsseldorf (DE)**

• **KRUSE, Daniela**
 **44577 Castrop-Rauxel (DE)**
• **KREIS, Peter**
 **44227 Dortmund (DE)**
• **SCHNITZER, Christian**
 **48249 Dülmen (DE)**
• **KOLEVA, Velichka**
 **45721 Haltern am See (DE)**
• **NISSEN, Felix**
 **48301 Nottuln (DE)**
• **BOECK, Florian**
 **48143 Münster (DE)**

(54) **MEMBRANE-BASED PROCESSES FOR PURIFICATION OF CASHEW NUT SHELL LIQUID**

(57)    The present disclosure in general relates to a process for reducing impurities, i.e. undesirable components, present in a cashew nut shell liquid (CNSL) using at least one selective nanofiltration membrane. It further provides new product mixtures from natural origin, comprising cardol and cardanol, that could not be obtained with processes of the prior art.

EP 3 251 737 A1

**Description**

The present disclosure in general relates to a process for reducing impurities, i.e. undesirable components, present in a cashew nut shell liquid (herein later "CNSL") using at least one selective nanofiltration membrane. It further provides new product mixtures from natural origin, comprising cardol and cardanol, that could not be obtained with processes of the prior art.

CNSL occurs as a greenish-yellow viscous liquid in the soft honeycomb of the shell of the cashew nut. The cashew nut is greenish-gray in color and is attached to the cashew apple. The nut has two walls or shells, the outer one being smooth and somewhat elastic and olive green in color before maturity. When the nut matures, the outside shell becomes grayish-brown in color. The inner shell is harder and must be cracked like the shells of other nuts. The honeycomb is housed between the inner and outer shells.

CNSL after isolation from the honeycomb, in particular industrial grade CNSL, is reddish-brown in color. CNSL constitutes about 20-25% of the weight of the cashew nut.

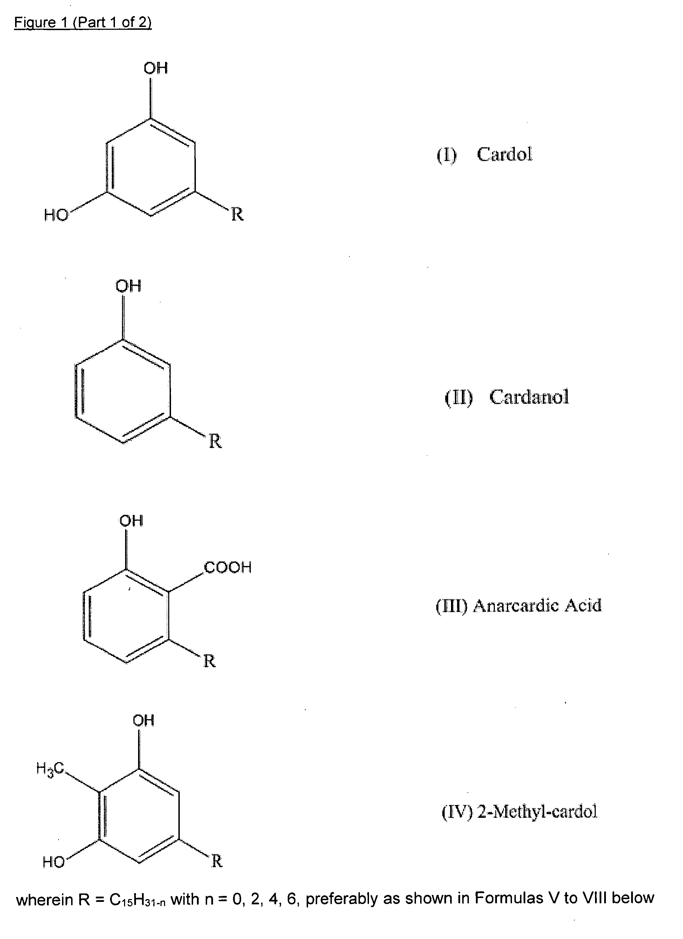

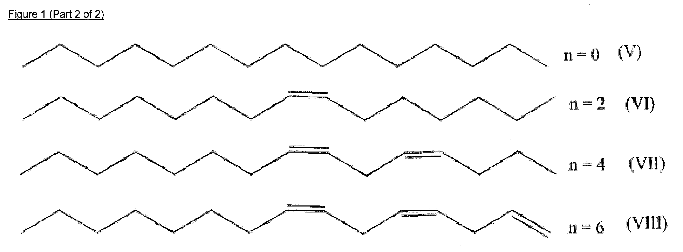

Many researchers have investigated the constitution of CNSL, e.g. A. R. R.Menon, J. Sci. Ind. Res. 44, 324 (1985), P. H. Gedam, Prog. Org. Coat. 14, 115 (1986) and J. H. P. Tyman, Chem. Soc. Rev. 8, 499-537 (1973). Accordingly naturally occurring CNSL contains mainly four components: cardol, cardanol, anacardic acid, and 2-methyl cardol (for details see Formulas (I) to (VIII) in Figure 1). Details of the compositions are disclosed in M.C. Lubi, Des. Monom. Polym., 2000, 3, 123, and the literature cited therein.

The composition of a crude CNSL, i.e. the contents of cardol, cardanol, 2-methylcardol, anacardic acid and impurities like polymers/oligomers, may vary in a wide range and strongly depends on the method used to the isolate crude of the CNSL from the honeycomb. The most important commercial process, i.e. the roasting process leads to a nearly quantitative decarboxylation of anacardic acid and also to the formation of up to 20 - 25% by weight of polymers and/ or oligomers (see J. H. P. Tyman, J. Am. Chem. Soc. 55, 663 (1978) and P. A. Mahanwar, Indian J. Technol. 3, 191- 193 (1996)).

Without being bond to any theory, it is postulated that the polymers/oligomers contribute to the intensive color and also to the high viscosity of crude CNSL. Both are disadvantageous for many potential applications of CNSL. As consequence many attempts have been made to purify the CNSL from polymers and other impurities. The commercial relevant process established today is a distillation process under vacuum at 150 to 200°C (see GB152925). Said distillation process is energy and thus cost intensive and causes significant losses of valuable compounds due to thermal decomposition and polymerization. In addition cardol and 2-methylcardol are separated during distillation from cardanol because of their boiling points, which are much higher than that of cardanol, what further reduces the yield of valuable compounds. At the end, the yield of valuable compounds from the CNSL, compared to its natural content in the nut shells is only 50 to 60%.

Attempts to purify CNSL by chromatography or extraction had no commercial relevance.

Beside of the coloration that occurs during isolation of CNSL, some authors reported that also purified CNSL underwent re-coloration during storage. It is believed that cardol might cause these problems. Thus, attempts have been made to completely separate cardol from the purified CNSL (see WO2006108545, WO2006108546 and GB2066820).

Beside of the fact that a complete separation of cardol further decreases the yield of valuable products, it is postulated, that in particular cardol and 2-methylcardol, due to their additional hydroxyl group, might be useful for specific applications because their polarity is higher than that of cardanol and they have an additional functional group in the molecule.

As consequence, there is still a strong need for a process that allows to obtain as much as possible of the valuable compounds of a CNSL in purified form. Preferably the desired products should have a much higher cardol and 2-methylcardol content than products obtained by prior art processes.

Object of the present invention was therefore to provide a process for purification of CNSL which does not have the drawbacks of the prior art processes or has such drawbacks only to a reduced extent.

The disclosed process should simplify and/or speed up the production of purified CNSL and potentially lead to purified CNSL compositions that cannot be obtained from natural sources with prior art processes.

Another problem was to provide a flexible process that allows to obtain different compositions of purified CNSL from natural sources, in particular with regard to the cardanol/cardol ratio.

In a special problem it should also be possible to obtain purified CNSL including high contents of anacardic acid from natural sources, what has not been possible with the prior processes, yet.

In a further special problem, the new process should allow to obtain purified CNSL with a low Gardner Color Scale (ASTM D 1544). In a first variant of this special problem, the process should allow to produce purified CNSL with low Gardner Color Scale for short term use, i.e. for applications wherein the purified CNSL is further processed shortly after its purification. In a second more challenging objective the process should allow a more long term use of the purified CNSL. In view of the second object it is a preferred problem that the Gardner Color Scale should not raise by more than 3 during long term use and even more preferred the Gardner Color Scale should not raise by more than 5 during very long term use. Said storage stability makes compositions comprising cardol and cardanol useful for new applications and in addition reduces the complexity and costs for storage and shipping.

Another special object of the invention is to reduce losses of cardol and/or 2-methylcardol after and during the purification process of the crude CNSL.

Also a special object of the present invention was to provide a more economic purification process compared to the prior art processes. Further problems not explicitly described above are obvious for one skilled in the art in view of the description, claims, examples and drawings of the present application.

The problems of the present invention are solved by a process according to claim 1 and by the product claims 13 and 14 of the present invention. They are further solved by a process or a product of the present invention, wherein at least one stabilizing acid and / or stabilizing oxygen scavenger is used to stabilize in particular Cardol and Cardanol. Preferred embodiments are claimed in dependent claims and/or are described in the subsequent description, examples and figures.

The present disclosure especially relates to a process to obtain purified CNSL. Said process allows to separate valuable components of the CNSL, in particular cardol, 2-methylcardol and cardanol, from impurities that cause coloration and/or increase the viscosity of the CNSL.

In the process according to claim 1 said valuable compounds can be obtained in high yields from natural sources and in form of product mixtures ratios of cardanol to cardol and/or 2-methylcardol, that cannot be obtained from natural sources with prior art processes.

A part of the inventive process is the isolation method of crude CNSL from the natural source. If an isolation methods is used that ensures, that the crude CNSL comprises significant amounts of anacardic acid, the process of the invention allows to obtain product mixtures that comprise significant amounts of anacardic acid as an additional valuable compound.

The process of the invention, thus allows to obtain new products mixtures for new applications.

Another advantage of the process of the invention is that the purification process is operated at low temperatures. Compared to conventional processes running at high temperatures, e.g. including vacuum distillation, the process of the invention saves a lot of energy and reduces operating and maintenance costs of the plant. This is because operation at high temperatures causes polymerization. Polymerization minimizes yields, but also increases maintenance costs because the equipment has to be shut down and purified regularly. The investment costs for the inventive process are also lower than for conventional processes.

Another unexpected advantage of the present invention was that high flow rates, up to at least 30 $kg/m^2h$ could be realized. This is beneficial for an economic process and allows to replace distillation processes.

As indicated before, the process of the invention allows high flexibility with regard to the product mix obtained. Depending on the choice of the isolation method used to obtain crude CNSL and of the details of the membrane separation process, different compositions of purified CNSL can be obtained if desired.

Another benefit of the process of the invention is its flexibility in view of the storage stability of the purified CNSL. Depending on the operating conditions during the purification step purified CNSL for short term use but also for long term use can be obtained.

The inventors further found out, that addition of a solvent to the composition comprising cardol and cardanol as well as stabilizing acids and / or stabilizing oxygen scavengers may improve the storage stability up to very long term (> 100 days) storage stability. Depending of the desired storage time and the requirements of re-coloration suppression, the present invention, thus, offers flexibility during commercial application. Use of a solvent slightly increases the costs, however, also improves the storage stability. Waive of solvent use on the other hand reduces costs and leads to products, that are at least for 25 days storage stable.

Using a combination of stabilizing acid and stabilizing oxygen scavenger, either by using two different both simultaneously or by combining both functions in one molecule, showed particular good results for very long term storage of the compositions of the present invention.

A next benefit of the present invention is, that it allows multiple ways of further processing of the purified CNSL. For example a second or third membrane separation step, optionally with different membranes, can be used to separate valuable compounds from each other. Combinations with different further processing methods are also possible.

Further advantages of the present invention, not described above, become obvious in view of the subsequent detailed description, examples, figures and claims.

It is to be understood that both the foregoing general description and the following detailed description are exemplary and explanatory only and are not restrictive of the disclosure, as claimed.

## BRIEF DESCRIPTION OF THE DRAWINGS

Figure 1 Structure formula of cardol, cardanol, 2-methylcardol and anacardic acid.
Figure 2 is a schematic of a dead-end membrane test system as used in example 1.
Figure 3 is a schematic of a cross-flow nanofiltration system, as used in ex. 2.
Figure 4 is a schematic of a diafiltration system
Figure 5 shows GPC analysis of example 1d.
Figure 6 shows GPC analysis of Ultralite 2023
Figure 7 shows GPC analysis of example 2a
Figure 8 shows an NMR analysis of the storage test of example 2b

**DESCRIPTION**

Particular aspects of the invention are described in greater detail below. The terms and definitions used in the present application and as clarified herein are intended to represent the meaning within the present disclosure. The patent and scientific literature referred to herein and referenced above is hereby incorporated by reference. The terms and definitions provided herein, if in conflict with terms and/or definitions incorporated by reference, are the terms and definitions that should be used for interpreting the present disclosure.

The singular forms "a," "an," and "the" include plural reference unless the context dictates otherwise.

The terms "approximately" and "about" mean to be nearly the same as a referenced number or value. As used herein, the terms "approximately" and "about" should be generally understood to encompass $\pm$ 30%, preferably $\pm$ 20%, particular preferred $\pm$ 10% and especially preferred $\pm$ 5% of a specified amount, frequency or value.

"Short term use" shall be understood with regard to the present invention, that during a storage time of a sample of the purified CNSL of up to 2 days, preferably up to 5 days, more preferred up to 10 days at room temperature (20°C) under air contact, the Gardner Color Scale of the sample increases by a maximum of 3 points, preferably by a maximum of 5 points.

"Long term use" in contrast thereto means, that the purified CNSL can be stored for a longer time compared to purified CNSL that is only suitable for short term use. "Long term use" shall be understood with regard to the present invention, that that during a storage time of a sample of the purified CNSL of up to 25 days or even longer at 20°C under air contact, the Gardner Color Scale of the sample increases by a maximum of 5 points, preferably by a maximum of 3 points.

"Very long term use" shall be understood with regard to the present invention, that that during a storage time of a sample of the purified CNSL of at least 100 days at 20°C under air contact, the Gardner Color Scale of the sample increases by a maximum of 5 points, preferably by a maximum of 3 points.

The term "isolation" means a process step, wherein a crude CNSL is obtained by separation (isolation) from the honeycomb of a cashew nut shell. Details will be discussed below.

"Refining" stands for process step wherein at least one valuable component of a CNSL is separated from at least one impurity or wherein said valuable component(s) is/are fractionated, and wherein a technique other than membrane separation is used the separation or fractionation.

"Purification" is to be understood as a process step that follows after isolation of a CNSL, wherein at least one impurity is separated from at least one valuable component of a CNSL or wherein at least one valuable component of a CNSL is fractionated and wherein membrane filtration is used for separation or fractionation.

The term "fractionation" as used in this disclosure for the purification step shall be understood to mean that the concentration of a given species in solution is different in the retentate and permeate solutions generated by applying the membrane separation process described herein. It will be understood by one skilled in the art that this means a given species could "fractionate" into either the permeate or retentate solutions, and furthermore that "fractionation" as defined here can incorporate enrichment, depletion, complete removal and complete capture of a species in either the retentate of permeate solutions.

The term "nanofiltration" as used in this disclosure shall be taken to mean a synthetic membrane that provides a nominal molecular weight cut-off in the range 150 g.mol$^{-1}$ to 1,500 g.mol$^{-1}$, where nominal molecular weight cut-off means the molecular weight at which the membrane provides a 90% rejection of a series of polystyrene oligomers (for example polystyrene polymer standards nominal Mp of 1,000 (part number PL2012-3010) and nominal Mp 580 (part number PL2012-2010) from Agilent Technologies) according to the method described in See Toh et al,

(Journal of Membrane Science, 291(1-2) (2007), 120-125). Nanofiltration membranes differ from ultrafiltration membranes (molecular weight cut-off in the range 2,000 Da to 2,000,000 Da) and microfiltration membranes (pore diameter 0.2 micron and larger).

The term "rejection" is defined by equation (1), where $C_{Pi}$ is the concentration of species i in the permeate solution, "permeate" being the liquid which has passed through the membrane, and $C_{Ri}$ is the concentration of species i in the retentate solution, "retentate" being the liquid which has not passed through the membrane. It will be understood by one skilled in the art that a rejection of 0% implies that the membrane offers no separation of species i, and a rejection of 100% implies that species i is completely retained. It will be further recognized by one skilled in the art that provide the rejection of species A does not equal the rejection of species B then the species can be fractionated, as defined herein.

$$R_i = \left(1 - \frac{C_{Pi}}{C_{Ri}}\right) \times 100\%$$

The terms "cashew nut shell liquid" and "CNSL" are used synonymously in the present invention. "CNSL" as a generic term covers different types/grades of CNSL that are obtained and/or used in the present invention as starting material or intermediate product or end product. Examples are a liquid, herein after called "natural CNSL", that occurs as a greenish-yellow viscous liquid in the soft honeycomb of the shell of the cashew or the liquid that is obtain when isolating the natural CNSL and purifying the same. Further examples are described in more detail below.

"Natural CNSL" shall mean greenish-yellow viscous liquid in the soft honeycomb of the shell of the cashew nut. It usually comprises valuable compounds, preferably cardol, cardanol, anacardic acid, and 2-methyl cardol. In addition it may comprise impurities, for example sulphides, nitrogenous material, minerals, polymers and compounds that cause its color.

The term "crude CNSL" in the following is used for a CNSL obtained after isolation of the natural CNSL from the honeycomb of the cashew nut. The isolation can be done by different methods and in one or more steps, as will be described in more detail below. Crude CNSL may, depending on the isolation method, comprise all or only some of above mentioned valuable components or impurities comprised in the natural CNSL. Preferably it comprises one or more components selected from the group consisting of cardol, cardanol, anacardic acid, and 2-methyl cardol. Crude CNSL may roughly be classified in two groups.

One group is "cold isolated CNSL" comprising as main components anacardic acid as well as cardol, cardanol and 2-methylcardol and in addition thereto oligomeric or polymeric compounds, especially those that are formed from cardanol, cardol or 2-methylcardol during the isolation of crude CNSL.

The other one is "technical CNSL" obtained by processes like roasting or hot-oil extraction that imply heat treatment of the CNSL. "Technical CNSL" comprises as main component cardanol, 2-methylcardol and in addition cardol. It further comprises polymeric materials that are mainly formed from cardanol, cardol or 2-methylcardol during heat exposure.

The term "purified CNSL" is used to characterize a CNSL that is obtained after purification and optionally refining of a crude CNSL. As consequence, "purification" or "purifying" differs from "isolation" because an already isolated crude CNSL is used, directly or after intermediate steps, as raw material for the purification. "Purification" in the sense of the present invention is done in one or more process steps wherein at least one process step is a nanofiltration. Before or after the nanofiltration, preferably before, other purification steps, preferably another nanofiltration or an ultrafiltration may be conducted. It is also possible that the process of the invention comprises one or more refining steps, before or after the nanofiltration, preferably before, wherein impurities like sulphides, nitrogenous material and minerals are separated.

"Valuable components" or "valuable compounds" stands for "cardol", "cardanol", "anacardic acid" and "2-methylcardol".

Each of the terms "cardol", "cardanol", "anacardic acid" and "2-methylcardol" is a generic term for a product family, that in each case comprise derivatives of the general formulas (I) to (IV) wherein "R" may vary and may preferably be selected from one or more radicals according to formulas (V) to (VIII) (see Figure 1). It is possible that only one or two or three or all four types of "R" are comprised.

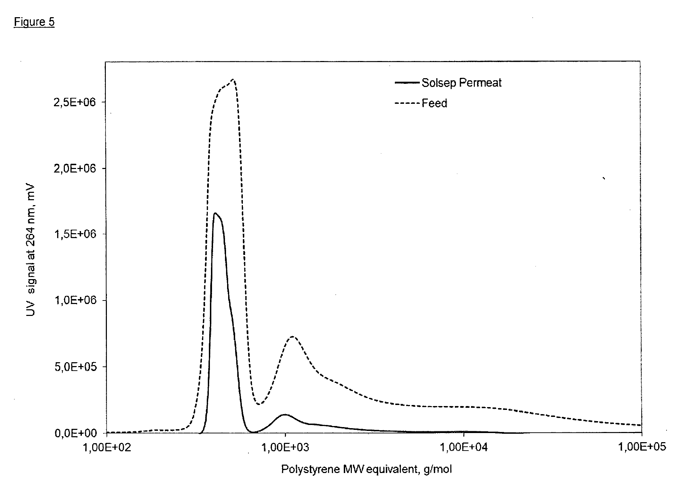

The term "oligomers and polymers" in the following is used for substances composed of molecules with large molecular weight Mp, preferably in the range of from 700 to 100.000 g / mol if determined with the GPC method described in the analytic methods section further below (see also Figures 5 to 7 of the present invention), and that are composed of repeating structural units, or monomers, connected by covalent chemical bonds. It stands for oligomers and polymers that are naturally occurring in the cashew nut or the cashew nut shells or the CNSL or that are formed during storage or heat treatment of cashew nuts or the cashew nut shells or the CNSL.

The terms "dilution" and "solution" are used synonymously in the present application. As will be explained in more detail below, it has some advantages to mix crude CNSL with at least one miscible solvent before membrane separation takes place and/or during storage of the purified CNSL. Depending on the desired effect different amounts of solvent can be added so that in one case one could speak of a dilution and in another of a solution. The borderline between solution and dilution might be fluent. Thus, for the present invention it does not matter whether a dilution or a solution is used, a man skilled in the art will chose the amount of solvent based on the desired effect to be achieved.

As mentioned before, different techniques are known to obtain crude CNSL from cashew nut shells. The most important commercial method implies heat treatment of the cashew nuts or cashew nut shells at temperatures above 150°C. One of these methods is the "hot oil bath method" or other extraction methods with hot oil. In the hot oil bath method usually the raw nuts are passed through a bath of hot CNSL, that is used as "oil", at preferably around 180-200°C. The outer part of the shell bursts open and releases CNSL. About 50% of the CNSL is thus recovered. This process makes the decortication of nuts easy, without adversely affecting the quality of the kernels. Improvements over this basic technique include initial surface wetting and dipping in water at 20-25°C and subsequent steam treatment prior to exposure to the hot CNSL bath. The excess moisture content of 7-10% of the weight of the nuts causes the cells to burst, with the result that the oil oozes into the bath. Another 20% of the CNSL could be extracted by passing the spent shells through an expeller and the rest by a solvent extraction technique. The expeller oil can be upgraded by acid washing followed by centrifugation and heating.

Another important isolation method comprising heat treatment is the "roasting method". Sudden exposure of the cashew nuts to high temperature from ambient is the basis of the method. The shells are charred during this process, producing an explosive pressure in the cellular structure which forces the liquid out of the shell. One variation of this method is abrading at 100-300°C for about 1 h and subsequent roasting at 400-700°C in an inert atmosphere. In yet another alternative, the nuts are first abraded and then treated with moisture and heated in an infrared oven. Finally they pass through a high-frequency electric field, where the liquid flows out. Often this technique is employed in conjunction with an expeller, where the CNSL is expelled from the shells to an extent of 90%.

Isolation processes comprising heat treatment are well known in the art. Further details can be found in Rodrigues F.H.A., Polimeros, Vol. 21, No. 2, 156-160, 2011 and the literature cited therein. These "hot isolation methods" lead to so called "technical CNSL" which is characterized by a high cardanol content in combination with a very low anacardic acid content and a significant content of polymeric and oligomeric materials. This is because anacardic acid is decarboxylized during heat treatment and transformed into cardanol. Typically "technical CNSL comprises 60 to 95% by weight cardanol, 4 to 19% by weight cardol, 1 to 2% by weight of anacardic acid, 0.3 to 10% by weight of polymeric material and 1.2 to 4.1% of 2-methylcardol. The percentage values given before in each case sum up to 100%. For details to the composition of technical CNSL reference is made to Mazzetto, G. - Quim. Nova, 32, p.732 (2009); Kumar, P. P., J. Agric. Food Chem., 50, p.4705 (2002) and Ikeda, R., Polymer, 43, p.3475 (2002). Even more details can be found in Lubi M.C., Des. Mon. Pol., Vol. 3, No. 2, 123, 2000 and the literature cited therein. Some technical CNSL comprises up to 25% by weight of polymeric and/or oligomeric material.

Cold isolation methods avoid heat exposure of the cashew nuts or cashew nut shells to temperatures above 150°C. One important method is to press out cashew nuts or cashew nut shells, preferably at ambient temperature. Another is to extract cashew nuts or cashew nut shells with organic solvents. Crude CNSL obtained from cold isolation methods usually comprises significant amounts of anacardic acid and therefore differs completely from technical CNSL as described before. Typically crude CNSL obtained by the cold isolation method comprises anacardic acid (46-65%); cardol (15-31%); cardanol (10-22%) and traces of methyl-cardol. The percentage values given before in each case sum up to 100%. For details to crude CNSL obtained by cold isolation methods reference is made to Rodrigues F.H.A., Polimeros, Vol. 21, No. 2, 156-160, 2011 and the literature cited therein.

Cold isolation methods as well as high temperature isolation methods are well known in the art. In particular after storage also the low temperature CNSL gets a red to brown color and comprises impurities like polymeric materials.

Depending on the desired end product technical CNSL or CNSL obtained by cold isolation methods can be used as raw material for the purification steps of the present invention.

In detail the present invention relates to a process for separating valuable components from impurities in a cashew nut shell liquid (CNSL) to obtain purified CNSL, comprising the following steps:

(i) providing a crude CNSL or a solution comprising a crude CNSL and at least one miscible organic solvent by isolation of the crude CNSL from the honeycomb of the shell of a cashew nut and optionally mixing the isolated CNSL with at least one miscible solvent,
(ii) optionally pre-purifying and/or refining the crude CNSL or the solution comprising crude CNSL,
(iii) providing a selectively permeable nanofiltration membrane having a first surface and a second surface;
(iv) separating the crude CNSL or the solution comprising crude CNSL from step (i) or (ii) by transferring one or more of its components from the first surface to the second surface across the membrane through contacting the crude CNSL or the solution comprising crude CNSL from step (i) or (ii) with the first surface, so that the components of the crude CNSL or the solution comprising crude CNSL from step (i) or (ii)l n contact with the first surface of the membrane form a retentate and the components of the crude CNSL or the solution comprising crude CNSL from step (i) or (ii)contacting the second surface form a permeate,

The process of the invention is further characterized in that
the pressure at the first surface of the membrane is higher than the pressure at the second surface, and
the crude CNSL or the solution comprising crude CNSL used in step (iv) comprises as valuable compounds cardol and/or cardanol and optionally one or two compound(s) selected from the group consisting of 2-methylcardol and anacardic acid; it further comprises at least one, preferably two or more impurities selected from the group consisting of polymers, oligomers and residues from cashew nut shells and

at least one, preferably two, more preferred three, valuable compound(s) is/are enriched and at least one, preferably two, more preferred three, impurity(s) is/are depleted in the permeate compared to the crude CNSL.

"Enriched valuable compound in the permeate" means, that the ratio of at least one valuable compound to at least one impurity in the permeate is higher than the ratio of both components in the crude CNSL. Analogously "depleted impurity" means that, the ratio of at least one impurity compared to at least one valuable compound in the permeate lower than the ratio of both components in the crude CNSL.

In step (i) crude CNSL is preferably isolated from whole cashew nuts and/or only the shells of cashew nuts as natural source by one of the methods described before. Preferred methods are cold isolation methods, in particular extraction or pressing out. Even more preferred methods are roasting and the hot oil methods. If a solution comprising crude CNSL is to be obtained in step (i) the solution may be either directly obtained during the isolation, for example if the isolation comprises an extraction with a miscible solvent, or the CNSL obtained after the isolation may be mixed with a miscible solvent.

The cashew nuts can be pre-treated before the isolation process starts, i.e. by cutting the cashew nuts or by separation of cores and shells of the cashew nuts or by cracking (opening) the outset shell of the cashew nuts or by wetting the cashew nuts and the like.

In an optional step (ii) the crude CNSL or the solution comprising crude CNSL may be pre-purified or refined. Details for refining are described above. Pre-purifying might preferably include one or more membrane separation and/or distillation steps conducted before step (iv) is executed. For example an ultrafiltration or microfiltration might be conducted in step (ii) and/or a filtration step with a different membrane compared to step (iv) might be conducted in step (ii).

Thereafter in steps (iii) and (iv) the main purification takes place, i.e. the valuable components of the crude CNSL or solution comprising the crude CNSL obtained after steps (i) or (ii) are separated from impurities, in particular from polymers and/or oligomers, which cause coloration and high viscosity. Other impurities might be sulfides, nitrogenous material, and minerals, residues from the cashew nut shells or the honeycombs or other impurities that got into the crude CNSL during its isolation. Thus, there might be "natural impurities", i.e. chemical species that are naturally occurring in the natural CNSL or other feedstock, but which are not desired species in the final purified CNSL. Typical non-limiting examples can include the compounds causing the greenish-yellow color of the natural CNSL and also the sulfides, nitrogenous material and minerals or sterols, lipophilic hormones etc. In contrast thereto are "synthetic impurities" which got into the crude CNSL during its isolation, e.g. extractables from the nut shells outer or inner walls, or into the natural CNSL during storage. Further non-limiting examples are polymers and / or oligomers formed during the isolation by heat exposure.

The concentration and composition of the impurities found in crude CNSL can vary. For example, a CNSL composition may vary based on geographical location where the plant is grown, harvesting season, etc. In some instances, the targeted impurities may be absent or below the detection limit, but if the CNSL is concentrated, the impurities may also be concentrated.

### *Membrane*

Suitable selective membranes for use according to step (ii) and (iii) of the present disclosure include polymeric and ceramic membranes, and mixed polymeric/inorganic membranes.

The at least one selective membrane used in the process of the present invention may be formed from any polymeric or ceramic material which provides a separating layer capable of fractionating the content of valuable components in the crude CNSL or separating the desired valuable components of the CNSL from at least one natural and/or synthetic impurity present in the natural and/or crude CNSL. For example, the at least one selective membrane may be formed from or comprise a material chosen from polymeric materials suitable for fabricating nanofiltration membranes, including preferably polyethylene, polypropylene, polytetrafluoroethylene (PTFE), polyvinylidene difluoride (PVDF), polysulfone, polyethersulfone, polyacrylonitrile, polyamide, polyimide, polyamide-imide, polyetherimide, cellulose acetate, cellulose triacetate, polyaniline, polypyrrole, polyphenylene oxide (PPO), polyetheretherketone (PEEK), polymers with intrinsic microporosity (PIM), polybenzimidazole, and mixtures thereof. The at least one selective membrane can be made by any technique known to the art, including sintering, stretching, track etching, template leaching, interfacial polymerization, or phase inversion. In a preferred embodiment the at least one selective membrane may be crosslinked or treated so as to improve its stability in the reaction solvents. For example, by way of non-limiting example, the membranes described in GB2437519, the contents of which are incorporated herein by reference, may be used in this disclosure.

In a further preferred embodiment, the at least one selective membrane is a crosslinked or none crosslinked composite material comprising a support and a thin, selectively permeable layer. The thin, selectively permeable layer may, for example, be formed from or comprise a material chosen from modified polysiloxane based elastomers including polydimethylsiloxane (PDMS) based elastomers, polyoctylmethylsiloxane (POMS) based elastomers, ethylene-propylene diene (EPDM) based elastomers, polynorbornene based elastomers, polyoctenamer based elastomers, polyurethane based elastomers, butadiene and nitrile butadiene rubber based elastomers, natural rubber, butyl rubber based elastomers, polychloroprene (Neoprene) based elastomers, epichlorohydrin elastomers, polyacrylate elastomers, polyethylene, polypropylene, polytetrafluoroethylene (PTFE), polyvinylidene difluoride (PVDF) based elastomers, polyetherblock amides (PEBAX), polyurethane elastomers, crosslinked polyether, polyamide, polyamine, polyaniline, polypyrrole, and mixtures thereof.

In an especially preferred embodiment of the present invention at least one selective nanofiltration membrane or nanofiltration composite membrane is used comprising a polyimide, particular preferred is a polyimide subject to post-formation crosslinking and / or impregnation. Crosslinking and/or impregnation agents are known to a man skilled in the art. Preferred crosslinking and/or impregnation agents are those disclosed in disclosed in GB2437519. Particular preferred impregnating agents are low volatility compounds. Very particularly preferred are polyimides, preferably crosslinked and/or impregnated, according to GB2437519 and Soroko et al. (Journal of Membrane Science, 381(1-2) (2011), 152-162) the contents of which are incorporated herein by reference.

Preferred are silicone-coated organic solvent nanofiltration membranes especially on the basis of polyimide or polyacrylonitrile membranes. Particular preferred are silicone acrylate coated membranes. Most preferred are membranes as for example described in DE10 2009 047 351 or disclosed in international patent application WO2012010889, the contents of which are incorporated herein by reference, may be used in this disclosure. A screening of different types of membranes (see examples 1a to 1e) revealed, that especially when using silicone-coated organic solvent nanofiltration membranes excellent purification effects in combination with high flow rats could be achieved. Thus, in silicone coated membranes, preferably silicone coated polymer membranes and more preferred silicone coated polyimide or polyacrylonitrile membranes are most preferred for use in the process of the present invention, in particular in the first membrane separation step.

Without being bound to any theory, the inventors are of the opinion, that the silicone coating minimizes deposition of polymeric compounds on the membrane surface, thus minimizing fouling which leads to stable membrane performance over time.

In another embodiment, the at least one selective membrane is prepared from an inorganic material such as, for example, silicon carbide, silicon oxide, aluminum oxide, zirconium oxide, titanium oxide, and zeolites, using any technique known to those skilled in the art such as sintering, leaching, or sol-gel processing.

In a further embodiment, the at least one selective membrane comprises a polymer membrane with dispersed organic or inorganic matrices in the form of powdered solids present at amounts up to 20 wt% of the polymer membrane. Carbon molecular sieve matrices can be prepared by pyrolysis of any suitable material as described in U.S. Patent No. 6,585,802. Zeolites as described in U.S. Patent No. 6,755,900 may also be used as an inorganic matrix. Metal oxides, for example, titanium dioxide, zinc oxide, and silicon dioxide may be used, such as the materials available from Evonik Industries AG (Germany) under their AEROSIL and ADNANO trademarks. Mixed metal oxides such as mixtures of cerium, zirconium, and magnesium may also be used. In at least one embodiment, the matrices will be particles less than 1.0 micron in diameter, for example less than 0.1 microns in diameter, such as less than 0.01 microns in diameter.

It is particularly preferred in all embodiments of the present invention that at least one selective membrane having a molecular weight cut-off ranging from about 150 g.mol$^{-1}$ to about 1,500 g.mol$^{-1}$, preferably from about 200 g.mol$^{-1}$ to about 800 g.mol$^{-1}$ and particular 200 g.mol$^{-1}$ to less than or equal to 600 g.mol$^{-1}$ is used in step (iv) the process of the invention.

In a special embodiment two or more membranes respectively two or more membrane separation steps are comprised in the process of the invention, for example in steps (ii) and (iv). In this special embodiment it is particularly preferred that two different membranes having two different molecular weight cut-offs are used. Very particularly preferred at least one membrane is used having a molecular weight cut off between 400 g.mol$^{-1}$ and 5000 g.mol$^{-1}$, especially between 500 g.mol$^{-1}$ and 1000 g.mol$^{-1}$ and at least one other membrane with a different molecular weight cut-off between 150 g.mol$^{-1}$and 800 g.mol$^{-1}$, especially between 300 g.mol$^{-1}$ and 600 g.mol$^{-1}$ is used. That the ranges for the molecular weight cut-off for the two different membranes as mentioned before overlap does not mean that identical membranes are used in both steps, even though this might be possible. In contrast, it has to be understood that a membrane having a molecular weight cut-off of 400 g.mol$^{-1}$ can be combined with a membrane having a molecular weight cut-off of 600 g.mol$^{-1}$. It is also possible, that membranes with identical molecular weight cut offs but made from different materials are used, especially in steps (ii) and (iv).

### *Process for Reducing Impurities*

Some embodiments of the present disclosure relate to a process wherein at least one, preferably two, more preferred three, valuable compound selected(s) from the group consisting of cardol, cardanol, anacadic acid and 2-methylcardol, is/are enriched in the permeate of a membrane separation stage compared to the crude CNSL respectively crude CNSL solution. Additionally, some embodiments of the present disclosure relate to a process for fractionating at least one valuable component from a CNSL using at least one selective membrane

According to one preferred embodiment, separation of valuable compounds from impurities respectively fractionation thereof may be achieved through contacting the crude CNSL or a solution of the crude CNSL in at least one miscible organic solvent obtained after steps (i) or (ii) with at least one selective membrane that retains the undesirable impurities, i.e., in the form of a retentate, and allows permeation of the desired valuable compounds, i.e., in the form of a permeate. Content permeates through the membrane due to, e.g., a trans-membrane pressure. In at least one embodiment, the trans-membrane pressure in step (iv) ranges from 1 to 100 bar, preferably from 5 to 75 bar, particular preferred 10 to 60 bar.

Fractionation of a valuable compound may be achieved through contacting the crude or purified CNSL or a solution of the crude or purified CNSL in at least one miscible organic solvent with at least one selective membrane. The valuable compound is then fractionated with at least one species having a different concentration in the retentate solution than in the permeate solution. Content permeates through the membrane due to, e.g., a trans-membrane pressure. In at least one embodiment, the trans-membrane pressure in step (iv) ranges from 1 to 100 bar, preferably from 5 to 75 bar, particular preferred 10 to 60 bar.

According to a preferred embodiment, the crude CNSL is mixed in steps (i) or (ii) with an organic solvent to form a homogeneous solution of crude CNSL and solvent. The mixing may be achieved by any technique known to one skilled in the art, such as, for example, via static inline mixer, dynamic inline mixer, and/or mixing vessel containing a mechanical stirrer. Preferably, the solution after steps (i) or (ii) contains the crude CNSL in an amount ranging from about 1 to 99% v/v, particularly preferred from 50 to 95 % v/v, particularly preferred from 60 to 90 % v/v, even more preferred from 65 to 85 % v/v. The term "organic solvent" when applied to the solvents used to prepare the mixture of crude CNSL and organic solvent includes, for example, an organic liquid with molecular weight less than 300 Daltons. The term "solvent" includes a mixture of organic solvents, as well as a mixture of organic solvents and water.

Preferred examples are, solvents including aromatic hydrocarbons, aliphatic hydrocarbons, ketones, glycols, chlorinated solvents, esters, ethers, amines, nitriles, aldehydes, alcohols, phenols, amides, carboxylic acids, furans, sulfoxide, thioether, epoxides, lactones and dipolar aprotic solvents, and mixtures thereof and with water.

Other preferred examples are, solvents including toluene, xylene, benzene, styrene, anisole, chlorobenzene, dichlorobenzene, chloroform, dichloromethane, dichloroethane, methyl acetate, ethyl acetate, isopropyl acetate, butyl acetate, methyl ether ketone (MEK), methyl iso butyl ketone (MIBK), acetone, ethylene glycols, ethanol, methanol, isopropanol, propanol, butanol, pentane, hexane, heptane, petrolether, cyclohexane, dimethoxyethane, methyl tert butyl ether (MTBE), diethyl ether, adiponitrile, N,N dimethylformamide, dimethylsulfoxide, N,N dimethylacetamide, dioxane, nitromethane, nitrobenzene, pyridine, carbon disulfide, tetrahydrofuran, methyl-tetrahydrofuran, N-methyl pyrrolidone, N-ethyl pyrrolidone, acetonitrile, and mixtures thereof and with water.

Particular preferred solvents are selected from the group consisting of methanol, ethanol, isopropanol, ethyl acetate, acetone, pentane, hexane, heptane, petrolether, ethylene glycols, and mixtures thereof and with water.

In one aspect, the present disclosure provides a process for reducing natural and / or synthetic impurities, preferably oligomers and polymers, more preferred polymers formed from valuable components, present in a natural or crude CNSL by applying membrane nanofiltration, wherein the process comprises the steps of: (i), (iii) and (iv) and optionally (ii) as described above, and. wherein the membrane(s) is/are selectively permeable membrane(s) such that the membrane rejection ($R_{Imp}$) of the impurities is greater than the rejection ($R_{Val}$) of the valuable components.

The disclosed method can also be used to fractionate at least one valuable component using the disclosed selective nanofiltration membranes, resulting in the formation of a concentrate comprising the at least one valuable component at a higher concentration than in the crude CNSL feedstock. The fractionation is carried out by applying the steps of: (i)), (iii) and (iv) and optionally (ii) as described above, and is characterized in that in step (iv) the one or more components to be fractionated from the other components of the crude CNSL are separated by passing components from the first surface to the second surface across at least one nanofiltration membrane by contacting crude CNSL or a solution of the crude CNSL in at least one miscible organic solvent with the first surface, wherein the pressure at the first surface is greater than the pressure at the second surface, and wherein the nanofiltration membrane(s) is/are selectively permeable membrane(s) such that the membrane rejection ($R_{Comp}$) of the components to be fractionated does not equal the rejection ($R_{Vall}$) of the other valuable components.

In a further embodiment, the process of the invention comprises subjecting the retentate and/or the permeate obtained after step (iv) to at least one additional processing step, preferably at least one further membrane separation step.

In a particular preferred embodiment, the process can be applied in multiple stages. By way of non-limiting example, a second stage further comprises providing the retentate or permeate from a first filtration; passing the first retentate or permeate solution across at least one further selective membrane, preferably also a nanofiltration membrane, wherein a second retentate and a second permeate are formed wherein further impurity reduction has been carried out.

In one embodiment, the crude CNSL or crude CNSL solution is contacted with at least one surface of at least one selective membrane, for instance, two or three selective membranes.

By way of non-limiting example, the crude CNSL or crude CNSL solution may first be contacted with one surface of the first selective membrane to remove impurities that, then the permeate comprising the valuable compound content from the first selective membrane is contacted with a first surface of a second selective membrane to remove impurities that permeated through the first membrane. The selected first and second membranes may be the same, or the selected membranes may be different in order to effect permeation of different impurities with the different membranes. It will be understood by one skilled in the art that contacting the CNSL or CNSL solution with three or more selective membranes may be necessary to provide the desired product.

In a further embodiment, the crude CNSL or crude CNSL solution may be contacted with a first surface of a first selective membrane to generate a retentate comprising the valuable compound content, too. The retentate may contain sufficient concentration of the valuable compounds that the retentate solution from the first selective membrane is then contacted with the first surface of a second selective membrane to generate a further permeate comprising the valuable compound content and a retentate stream containing the impurities. It will be clear to one skilled in the art that by processing the first retentate solution with a second membrane, the yield of the desirable valuable compounds will be increased. Furthermore, it will be clear to one skilled in the art that process configurations including both a series of selective membranes processing the CNSL or CNSL solution and retentate and a series of selective membranes processing the permeate solution from any other selective membranes are feasible.

Thus, in at least one embodiment, the process disclosed herein further comprises (v) mixing the retentate of stage (iv) with an organic solvent to form a retentate solution; (vi) passing said retentate solution across at least one selective membrane, wherein a further permeate forms comprising valuable components content, and a further retentate forms comprising at least one impurity.

In yet another embodiment, the process disclosed herein further comprises (v) mixing the permeate of stage (iv) with an organic solvent to form a permeate solution; and (vi) passing the permeate solution across the at least one selective membrane, wherein a further permeate forms comprising valuable compounds content, and a further retentate forms comprising at least one impurity.

In at least one embodiment, repetition of the process of mixing, passing, and removing may continue for a period of time ranging from about 10 minutes to about twenty hours. For example, in one embodiment, repeating the process of mixing, passing, and removing continues for a period of time ranging from about 30 minutes to about five hours.

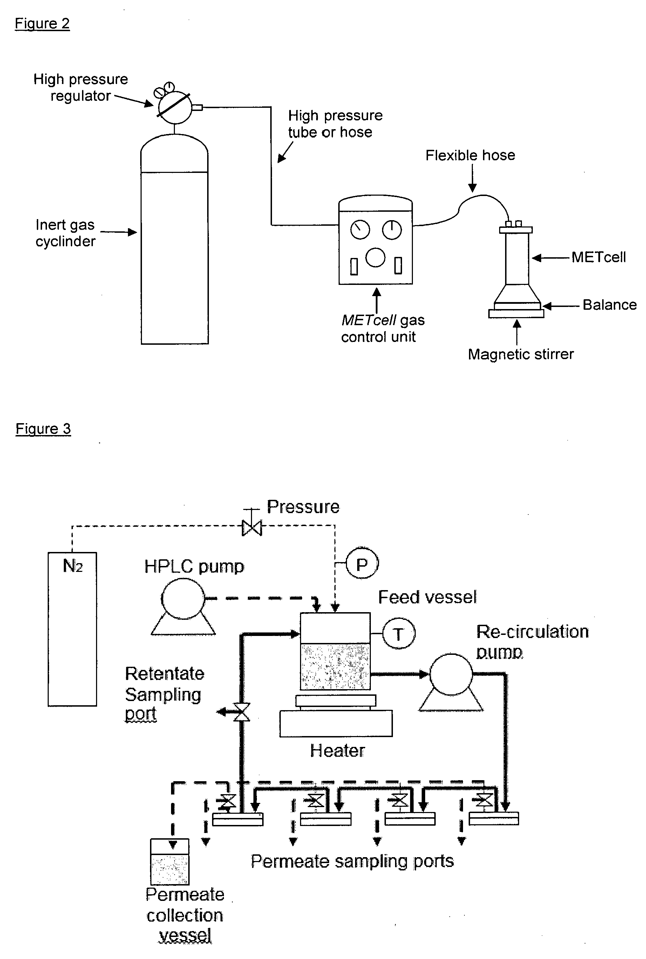

In a preferred embodiment, the crude CNSL or the diluted crude CNSL is contacted with the first surface of the membrane by flowing tit tangentially across the first surface. This is commonly known as "cross flow" filtration or "tangential flow" filtration (see Figure 3). As a result, the impurity content is retained as the retentate, and at least one valuable compound permeates through the at least one selective membrane. When tangential flow filtration is used to pass the solution across the surface of at least one selective membrane, the process may comprise a linear velocity at the membrane surface ranging from about 0.1 m/s to about 5 m/s, such as, for example, from about 0.5 m/s to about 3 m/s.

In the process disclosed herein, diafiltration is also a preferred alternative to enhance the removal of impurities from the CNSL. Diafiltration is known to those skilled in the art and is the process whereby fresh solvent is added to a solution undergoing filtration to enhance the quantity of lower molecular weight species that permeate through the membrane. Diafiltration is a liquid filtration process in which a feed liquid containing at least two solutes is in contact with a membrane and is pressurized so that some fraction of the liquid passes through the membrane, wherein at least one solute has a higher rejection on the membrane than at least one other solute. Additional liquid is fed to the pressurized side of the membrane to make up for the liquid permeating through the membrane. The ratios between the concentration of the more highly retained solute and the concentration of the less retained solute in the permeate and retentate varies dynamically, increasing in the retentate and decreasing in the permeate. Thus, in at least one embodiment, the passing of the solution across the at least one selective membrane comprises diafiltration.

A very particular preferred method for the present invention is a combination of cross-flow and diafiltration. Compared to other known processes like dead-end filtration, the preferred process of the present invention provides several advantages like: less fouling; less material loss, longer life time of the apparatus. In sum a higher efficiency can be achieved.

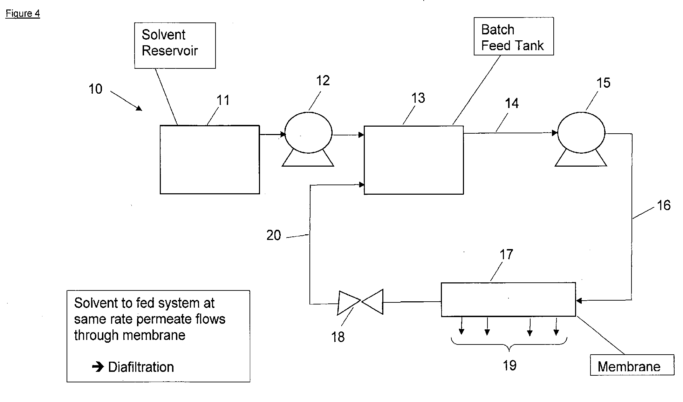

For example, a diafiltration system is illustrated in Figure 4. First a batch of the crude CNSL or solution of the crude CNSL to be processed is fed into tank 13. Pump 15 is then used to circulate the crude CNSL or solution of the crude CNSL (14 and 16) to a membrane module housing (17) in which a module containing a suitable membrane for the separation is located. The driving force for the separation is generated by a back-pressure valve (18), which provides a filtration pressure that maintains a trans-membrane pressure difference that allows a portion of the feed fluid to transport through the membrane to generate a permeate stream (19) and a retentate stream (20). The retentate stream (20) is returned to the feed tank (13). In order to maintain a constant volume in this system, solvent is fed from reservoir 11 to feed tank 13 by pump 12 at the same rate as liquid is permeating through the membrane (19). By applying this process, valuable components are flushed through the membrane whilst the impurities are retained, thus generating a purified CNSL.

In at least one embodiment, solvent content in the permeate material is optionally recovered. The recovered solvent content may then be reused to dissolve the crude CNSL. By way of non-limiting example, the solvent may be recovered by a thermal process such as flash evaporation or thin-film evaporation, or it may be recovered using a membrane filtration process where the valuable components are retained by the filtration membrane. In addition, in at least one embodiment, the permeate material is subjected to additional processing to recover desired components. Subsequent recovery of the desired compounds may be carried out by, for example, molecular distillation, short path evaporation, or chromatographic processes, such as HPLC (high pressure liquid chromatography) or supercritical chromatography, depending on the application.

In particular anacardic acid, cardol and 2-methylcardol are known to be vulnerable to thermal degradation or heat induced polymerization. Compared to other known methods for the removal of natural and synthetic impurities, the method disclosed herein may be performed effectively at near-ambient or sub-ambient temperature conditions. Many of the other known methods of impurity removal involve higher temperatures, which may be harmful to the thermally-sensitive components, i.e. the thermally-sensitive components are converted to different chemical species which reduces their yield and may also change the organoleptic properties or efficacy of the purified CNSL. The process, in particular the nanofiltration process, of the present invention can preferably be carried out at about 0 to 80°C, more preferred 5 to 70°C, even more preferred 10 to 60°C and most preferred 20 to 50 °C.

In the purifying process of the present invention at least one valuable compound is enriched in the permeate of a membrane in step (iv), wherein enriched means that the ratio of a valuable compound to at least one impurity is shifted to the side of the valuable compound, i.e. is increased. Preferably the ratio of cardanol and / or cardol to at least one polymeric compound, determined by GPC or NMR, in the permeate of a membrane separation is increased by at least 10%, more preferred by at least 30%, even preferred by at least 50%, particular preferred by more than 70% and most preferred by more than 90%. In the ideal case the polymeric compounds are separated completely, i.e. by 100%.

The inventors of the present invention have found out, that using crude CNSL as raw material without dilution is a preferred method to obtain purified CNSL applicable for short term use. As will be shown in the examples, very good purification effect can be achieved with un-diluted crude CNSL. It has, however, been observed, that the purified CNSL in such cases tends to re-coloration after storage for 2 days at room temperature under air.

In contrast thereto, the inventors found out, that using diluted crude CNSL as feedstock for the membrane separation, a very good purification effect can be achieved and in addition the purified CNSL is suitable for long-term use. Without being bond to any theory the inventors believe that in particular cardol tends to polymerize or to form chinones if stored under air. Dilution of the crude CNSL reduces the probability that two cardol molecules met each other to form the polymer chain. It is, thus preferred to dilute the CNSL feedstock for the membrane filtration with 1 to 99% by weight, preferably with 5 to 50% by weight, more preferred with 10 to 40% by weight and most preferred with 15 to 535% by weight of at least one miscible organic solvent to obtain a homogeneous mixture. "% by weight" in the sentence before means "% by weight of the total weight of the mixture of solvent and CNSL". Other, also preferred, alternatives to obtain purified CNSL for long term use are dilution of the purified CNSL with a miscible solvent for or during storage and/or storing the purified CNSL under inert conditions. Combinations of the methods described in this and the paragraphs before are also covered by the present invention. It is, thus preferred to store the purified CNSL in form of a diluted mixture of valuable compounds and at least one miscible organic solvent, wherein the content of the valuable compounds in sum is in the range of from 1 to 99% by weight, preferably with 50 to 95% by weight, more preferred with 60 to 90% by weight and most preferred with 65 to 85 by weight to obtain a homogeneous mixture. "% by weight" in the sentence before means "% by weight of the total weight of the total composition including valuable compounds and solvent". Solvents as described above can be used.

Beside of the increased storage stability it has shown that with diluted CNSL feedstocks higher flow rates were achieved. This is attributed to a lower viscosity of the feedstock. The higher flow rate, however, is accompanied with an increased amount of feed stock that has to be processed. In the diluted but also in the non-diluted cases commercially acceptable flow rates were achieved. With this knowledge, it is thus, easy for a man skilled in the art to select an appropriate solvent or solvent mixture respectively an appropriate dilution rate to achieve the desired effect of use time and processing time.

If very long term use, i.e. stabilization of the purified CNSL for more than 100 days, is desired the inventors found out, that it is possible to add one or more a stabilizing agents, preferably selected from the group consisting acids or their salts, oxygen scavengers, phenothiazines, phenolic stabilizers and mixtures thereof, either in form of different molecules or combined in one molecule to the CNSL solution. As shown in the examples, the inventors tested several potential stabilizers and found, that acids or their salts, selected from the group consisting of

- substituted or unsubstituted, saturated or unsaturated $C_1$ to $C_{12}$-mono-, di, or tri-carboxylic acids, preferably acetic acid, acrylic acid, oxalic acid, malonic acid, succinic acid, glutaric acid, adipic acid, sebacic acid, dodecanedioic acid, citric acid especially preferred oxalic acid, acrylic acid, malonic acid and citric acid,
- substituted or unsubstituted $C_1$ to $C_6$-mercaptocarboxylic acids or $C_1$ to $C_6$-mercaptocdiarboxylic acids, preferably thioglycolic acid, mercaptosuccinic acid and 3-mercaptopropionic acid , especially preferred thioglycolic acid,

- substituted or unsubstituted aromatic carboxylic acids, preferably benzoic acid and anacardic acid, salicylic acid , especially preferred benzoic acid,
- polyacrylic acids or polyacrylic acids copolymers,
- mineral acids and their salts, preferably phosphoric acid, phosphorous acid ($H_3PO_3$), hydrophosphorous acid ($H_3PO_2$), polyphosphoric acid, sulfuric acid or sulfamic acid, and
- substituted or unsubstituted aromatic or aliphatic sulfonic acid and

oxygen scavengers, selected from the group consisting of

- phosphines, preferably phosphine, triethylphosphine, tributylphosphine, trihexylphosphine trioctylphosphine, triphenylphosphine especially preferred tributylphosphine and triphenylphosphine,
- phosphites, preferably trialkylphosphites like trimethylphosphite, triethylphosphite or triarylphosphites, e.g. triphenylphosphite, tris(nonylphenyl)phosphite, tris(2,4,di-tert-butylphenyl)phosphite, especially preferred arylphosphites e.g. triphenylphosphite, tris(nonylphenyl)phosphite,
- hydroxylamines, preferably hydroxylamine, diethylhydroxylamine and dibenzyl hydroxylamine, especially preferred diethylhydroxylamine,
- thioether, sulfites, diethyl sulfide, dimethyl sulfide, di-n-butyl sulfide, ethyl n-octyl sulfide, methyl ethyl sulfide, n-dodecyl methyl sulfide, distearyl thiodipropionate, dimyristyl thiodipropionate, ditridecyl thiodipropionate, especially preferred dimethyl sulfide, di-n-butyl sulfide,
- disulfides and polysulfides, preferably dimethylsulfides, di-tert-butyl polysulfide, di-tert-nonyl polysulfide, dioctadecyldisulfide, and
- substituted or unsubstituted, saturated or unsaturated $C_1$ to $C_{12}$-mercaptans, preferably ethanthiol, tertbutyl mercaptan and dodecanthiol, especially preferred dodecanthiol,

lead to very good stabilizing results. Combination of stabilizing acids or their salts and oxygen scavenger lead to even better results. Thus, use of stabilizing acids or their salts and oxygen scavenger or both in combination as stabilizing agent is most preferred.

As demonstrated in the examples below, the stabilizing agents ensure a storage stability of the inventive compositions at 20°C under air of up to 105 days or even longer while several typically known stabilizing agents like radical polymerization inhibitors did not have any comparable stabilizing effect or even improve coloration of the composition (see comparison examples in Tables 5 and 6).

The examples further show that molecules like thioglycolic acid, wherein an acid and an oxygen scavenging functionality are combined in one molecule show even better performance than substances having only one functionality in the molecule. It is thus, preferred, to use stabilizing agents comprises at least one acid and at least one oxygen scavenger either in form of separate molecules or in form of one molecule that combines both functionalities. Particular preferred is, that the ratio of acid(s) in sum to oxygen scavenger(s) in sum in the compositions of the invention is in the range of from 50 : 50 to 99 : 1, preferably of from 60 : 40 to 90 : 10 and even more preferred of from 70 : 30 to 80 : 20.

The examples also show, that storage tests with diluted CNSL and acids and/or oxygen scavengers showed the lowest Gardner Color Numbers and even Iodine numbers were measurable, compared to storage tests with undissolved CNSL and acids and/or oxygen scavengers. For very long term use, compositions of the invention comprising at least one solvent and at least one stabilizing agent, preferably at least one acid or acid salt and/or oxygen scavenger, are preferred.

Preferably the stabilizing agent(s) is/are added in the process of the invention after step (iv), i.e. to the purified CNSL or purified CNSL solution. It is, however, also possible to add the stabilizing agents(s) before or during any one of steps (i) to (iv). It is also possible to add it in one or several portion(s) before, during or after any one of steps (i) to (iv). Most preferred, the stabilizing agents(s) is/ are added after step (iv).

As mentioned before, natural CNSL or crude CNSL may comprise different types of impurities. In a preferred alternative of the present invention most of, respectively all impurities are separated from the valuable compounds by membrane separation. It is, however, also possible to include one or more refining step(s) into the process, for example in step (ii) or in a step after step (iv). Non-limiting examples of refining steps are extraction of at least one impurity or of the valuable compounds from the CNSL, chemical and / or acid treatment of the CNSL, washing, crystallization, distillation adsorption of impurities, e.g. with active carbon, chromatography, high pressure liquid chromatography, supercritical chromatography, or resin or ion exchange adsorption, depending on the application etc.. These methods are generally known to a man skilled in the art and for example described in Lubi M.C., Des. Mon. Pol., Vol.3, No. 2, 123, 2000 and the literature cited therein. Refining steps can be done at different stages of the inventive process, i.e. with the natural CNSL, the crude CNSL and / or the purified CNSL. It is, however, preferred to conduct at least one refining step before the first membrane separation step, in particular preferred with the crude CNSL. It is also preferred to use only refining steps that are conducted at temperatures below 80°C.

It will be understood by one skilled in the art that the disclosed method also relates to a process for reducing the amount of at least one valuable component in a CNSL composition thus generating a concentrate of the at least one component. The resulting concentrate(s) comprising at least one desired component may be used directly as a product in its own right, or it can be used as an intermediate that is subject to further separation processes, such as for instance chromatographic methods or crystallization.

It will be understood by one skilled in the art that the embodiments described above are not limiting examples and within the spirit of this disclosure the embodiments can also be combined and applied in different sequences to achieve a desired product.

### *Resulting Composition(s)*

The present disclosure also relates to compositions preferably resulting from the process disclosed herein. Such compositions may not be obtainable from natural sources by processes of the prior art.

For instance, the disclosed process produces purified CNSL form a natural source, i.e. cashew nuts. Said products comprise cardanol but in addition also cardol and optionally anacardic acid in high contents. Simultaneously the contents of polymers and oligomers are very low. As consequence, they show a very low Gardner Color Scale.

In a first preferred embodiment, the products of the invention are obtainable by the process of the invention from a crude CNSL that is isolated from the cashew nuts by a cold isolation method. Compared to prior art products as for example disclosed in EP 2 578 670, those products are nearly free or free of polymers and oligomers, i.e. have a low Gardner Color Scale, but simultaneously comprise high amounts of anacardic acid, cardol and cardanol. The products of EP 2 578 670 are CNSL liquids that are isolated by a cold isolation method, too, but they are stabilized directly after isolation or used without any purification or refining. Impurities, in particular polymers and oligomers are still comprised in the CNSL of EP 2 578 670. As consequence, the products of EP 2 578 670 can only be used as animal feed, where the color of the CNSL is not important, while the products of the first preferred embodiment of the present invention are usable in applications with high optical requirements, details see further below.

A first preferred product according to the present invention is characterized in that all valuable components are of natural origin and that it comprises a mixture of:

a) Anacadic Acid: 35 - 80 % by weight of the mixture; preferably 40 - 75 % by weight of the mixture, particular preferred 45 - 70 % by weight of the mixture

b) Cardol 5 - 40 % by weight of the mixture; preferably 10 - 35 % by weight of the mixture, particular preferred 10 - 30 % by weight of the mixture and most preferred 10 - 25 % by weight of the mixture

c) Cardanol 5 - 30 % by weight of the mixture, preferably 5 - 25 % by weight of the mixture, particular preferred 10 - 25 % by weight of the mixture

d) 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture

e) Polymers and oligomers 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture

wherein the amounts of components a) to e) are selected in a manner that the sum of components a) to e) in the total mixture of a) to e) calculates to 100 % by weight.

The first preferred product might consist only of the mixture of components a) to e). In that case the sum of 100% by weight of the mixture of components a) to e) is identical 100% by weight of the total composition. It is, however, also possible, that the first preferred product comprises additional components beside of the mixture of components a) to e). Preferred, non limiting, examples of such additional components are solvents and stabilizing agents as defined above. In that case the mixture of a) to e) represent only a part of the whole composition, i.e. even though the sum of a) to e) calculates to 100% by weight with regard to the mixture of a) to e), said 100% by weight are only a portion of the 100 % by weight of the overall composition.

Components other than the mixture of a) to e) might be comprised in the first preferred product in sum in an amount of from 0.0005 to 95 % by weight, preferably of from 0.0001 to 90 % by weight, more preferred of from 0.001 to 80 % by weight and most preferred of from 0.01 to 70 % by weight, "% by weight" means "by weight of the total composition". The lower limit is preferably valid for cases where only stabilizers are comprised as additional component, while the upper limit might be reached for example if one or more solvents are added.

If one or more solvent(s) is/are comprised in the first preferred product their content in sum is of from 1 to 95 % by weight, preferably of from 10 to 90 % by weight, more preferred of from 20 to 80 % by weight and most preferred of from 30 to 70 % by weight, "% by weight" means "by weight of the total composition".

If one or more stabilizing agent(s) as defined above is/are present in the first preferred composition, they are preferably comprised in sum in an amount of from 20 to 0.0001 % by weight, preferably of from 10 to 0.0005 % by weight, more preferred of from 1 to 0.001 % by weight and most preferred of from 0.1 to 0.01 % by weight, "% by weight" means "by weight of the total composition".

The components of the first preferred composition as described above can be comprised in different ratios to each other. In any case, the amounts are selected from the ranges given above in a manner that the amounts of all components together calculate to 100 % by weight of the total composition.

In a second preferred embodiment, the products of the invention might be obtained by the process of the invention from a crude CNSL that is isolated from the cashew nuts by a hot isolation method, preferably by using technical CNSL as crude CNSL. Compared to prior art products the products of the invention are nearly free or free of polymers and oligomers and anacardic acid, i.e. have a low Gardner Color Scale, but simultaneously comprise high amounts of cardol and cardanol.

A second even more preferred product according to the present invention, thus, is characterized in that all valuable components are of natural origin and that it comprises a mixture of:

A. Anacadic Acid: 0 - 5 % by weight of the mixture, preferably 0,1 - 4 % by weight of the mixture, particular preferred 0,5 - 3 % by weight of the mixture, even more preferred 1 - 2 % by weight of the mixture

B. Cardol 2 - 35 % by weight of the mixture; preferably 3 - 30 % by weight of the mixture, particular preferred 4 - 25 % by weight of the mixture, most preferred 5 - 20 % by weight of the mixture

C. Cardanol 55 - 95 % by weight of the mixture, preferably 60 - 90 % by weight of the mixture, particular preferred 65 - 85 % by weight of the mixture

D. 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture

E. Polymers and oligomers 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture

wherein the amounts of components A) to E) are selected in a manner that the sum of components A) to E) in the total mixture of A) to E) calculates to 100 % by weight.

The second preferred product might consist only of the mixture of components A) to E). In that case the sum of 100% by weight of the mixture of components A) to E) is identical 100% by weight of the total composition. It is, however, also possible, that the second preferred product comprises additional components beside of the mixture of components A) to E). Preferred, non limiting, examples of such additional components are solvents and stabilizing agents as defined above. In that case the mixture of A) to E) is only a part of the whole composition, i.e. even though the sum of A) to E) calculates to 100% by weight with regard to the mixture of A) to E), said 100% by weight are only a portion of the 100 % by weight of the overall composition.

Components other than the mixture of A) to E) might be comprised in the second preferred product sum in an amount of from 0.0005 to 95 % by weight, preferably of from 0.0001 to 90 % by weight, more preferred of from 0.001 to 80 % by weight and most preferred of from 0.01 to 70 % by weight, "% by weight" means "by weight of the total composition". The lower limit is preferably valid for cases where only stabilizers are added as additional component, while the upper limit might be reached for example one or more solvents are added.

If one or more solvent(s) is/are comprised in the second preferred product their content in sum is of from 1 to 95 % by weight, preferably of from 10 to 90 % by weight, more preferred of from 20 to 80 % by weight and most preferred of from 30 to 70 % by weight, "% by weight" means "by weight of the total composition".

If one or more stabilizing agent(s) as defined above is/are present in the second preferred composition, they are preferably comprised in sum in an amount of from 20 to 0.0001 % by weight, preferably of from 10 to 0.0005 % by weight, more preferred of from 1 to 0.001 % by weight and most preferred of from 0.1 to 0.01 % by weight, "% by weight" means "by weight of the total composition".

The components of the second preferred composition as described above can be comprised in different ratios to each other. In any case, the amounts are selected from the ranges given above in a manner that the amounts of all components together calculate to 100 % by weight of the total composition.

The products of the invention are of natural origin and contain cardol and cardanol as well as optionally anacardic acid and 2-methyl cardol in high amounts. In addition they are nearly free or free of oligomers and polymers. It might be possible to obtain product mixtures comparable to those of the present invention via a synthetic route, i.e. by obtaining components of the mixture via chemical synthesis and then mixing the individual components together. Products obtained by such a process, however, are disadvantageous because their production is much more cost intensive and complex.

As mentioned before, the products of the present invention are nearly free or free of oligomers and polymers. In addition the term of use can flexibly be chosen from short term use up to very long term use in the stabilized version. Thus, in contrast to products as described for example in EP 2 578 670, they have a low Gardner Color Scale and can be used for applications with high optical requirements. Preferred field of use are: coatings, plastics household products, care products and oil additives. Particular preferred field of application are as dispersant, curing agent, cross linker, plasticizer, surfactant, emollient and lubricant.

The examples disclosed below are provided for a deeper understanding and clarification of the present invention. They, however, may not be construed to restrict the scope of the invention in any way

Analytic Methods

GPC

Gel-Permeation Chromatography (GPC) is carried out using a Shimadzu LC 20AT equipped with a Shimadzu SIL-20AC Auto Sampler, a Shimadzu DGU 20A5 Degasser, a Shimadzu SPD-20A UV/VIS Detector and a Shimadzu CTO-20AC COLUMN-Oven. The applied method is 35 °C, 1.00 mL/min and 264 nm wavelength with Agilent PLgel 5$\mu$m 50A as column and THF inhibitor free form Chromasolv Plus as eluent.

GARDNER COLOUR SCALE

The Gardner Color is measured with a LICO 200 from Hach-Lange. Eight reference solutions based on hydrochloric Potassium-hexachloroplatinat (IV) are used. The value of each solution is implemented in the software of the instrument. The measurement is performed in 11 mm round for wavelengths from 380 to 780 nm. Solvent or water are used as baseline. Measurements are done according to DIN EN ISO 4630-2.

FLOW RATE

The flow rate of the permeate is determined by measuring the mass of permeate over time. Therefore a Sartorius balance CPA 62025 is applied.

IODINE NUMBER

[0137] The Iodine Number is measured with a LICO 200 from Hach-Lange. Eight reference solutions based on hydrochloric Potassium-hexachloroplatinat (IV) are used. The value of each solution is implemented in the software of the instrument. The measurement is performed in 11 mm round for wavelengths from 380 to 780 nm. Solvent or water are used as baseline. Measurements are done according to DIN EN ISO 4630-2

CONTENT OF VALUABLE COMPONENTS RESPECTIVELY IMPURITIES

The content of valuable components respectively impurities were analyzed by the combination of NMR Spectroscopy and Gel Permeation Chromatography. Cardol, Cardanol, 2-Methylcardol , Anacadic Acid and solvent ratio were analyzed by $^1$H-NMR spectroscopy using a Bruker Advance 500 (500 MHz,). $CDCl_3$ as solvent and Dimethylterephthalate as internal standard. Ratio were calculated by the integration of the aromatic signals and the olefinic signals

The amount of oligomeric and polymeric compounds were analyzed via GPC using THF inhibitor free form Chromasolv Plus as eluent and Agilent PLgel 5$\mu$m 50A as column.

Sulfur and Nitrogen content were determined via elementar analysis with Elementar-Analysator EuroEA 3000 from Hekatech.

The examples provided below are intended for better understanding and clarification of the invention the may not be construed to limit the scope of the invention in any way.

EXAMPLES

**Example 1 - Membrane Screening experiments**

As crude CNSL a cashew nut shell liquid provided by Thao Nguyen Co., Vietnam, was used. The crude CNSL was obtained by the roasting method.

A dead-end METcell filtration cell (Evonik Membrane Extraction Technology Ltd, UK) containing a coupon of different membranes, was used for this experiment - see Figure 2 for a schematic diagram of the METcell dead-end filtration system. Details of the tested membranes see Table 1. The membranes were made ready for use by filtering 100 ml of isopropanolat ambient temperature and 10 bar filtration pressure to remove the conditioning agent (preservative) from the membranes.

Once the membrane was ready, the filtration cell was emptied and refilled with 250 mL of crude CNSL. The crude CNSL was filtered at a temperature and pressure given in Table 2. Every two hours samples of the permeate were collected and their Gardner Color Scale was analyzed. In addition the flow rates through the membranes were measured.

Table 1 Membranes Used

| Example | Membrane | Source and Description of Membrane |
|---|---|---|
| 1a | PuraMem®S600 | PuraMem® S600 is a silicone acrylate-coated polyimide membrane prepared according to DE10 2009 047 351 with a nominal molecular weight cut-off of 600 $g.mol^{-1}$. The membrane is commercially available from Evonik Membrane Extraction Technology Ltd, UK |
| 1b | PuraMem®280 | PuraMem® 280 is a polyimide membrane with a nominal molecular weight cut-off of 280 $g.mol^{-1}$, commercially available from Evonik Membrane Extraction Technology Ltd, UK |
| 1c | ONF-2 | ONF-2 is an organophilic nanofiltration membrane with an active layer made of polydimethylsiloxane obtainable from GMT/Borsig Its molecular weight cut-off of~380 $g.mol^{-1}$.(PS in Toluol) |
| 1d | SolSep NF030705 | SolSep NF030705 is an organophilic nanofiltration membrane with an unknown active layer from Solsep BV, NL. Its molecular weight cut-off of~500 $g.mol^{-1}$ .(PS in Heptane) |

TABLE 2 test conditions

| Example | Operating Temperature [°C] | $N_2$-Pressure [bar] |
|---|---|---|
| 1a | 50 | 50 |
| 1b | 50 | 50 |
| 1c | 50 | 60 |
| 1d | 60 | 40 |

TABLE 3 test results

| Example | Flow Rate [$kg/m^2h$] | Gardner Color Scale of Sample No. | | | | | | | | | |
|---|---|---|---|---|---|---|---|---|---|---|---|
| | | Feed | 1 | 2 | 3 | 4 | 5 | 6 | 7 | 8 | 9 | 10 |
| 1a | 0.5 | >18 | N.D. | 11.3 | 11.7 | 8.5 | 8.6 | 9.9 | 8.3 | 8.0 | 17.7[1] | 11.3[1] |
| 1b | 1.0 | >18 | 10.9 | 10.5 | 9.4 | 8.9 | N.D. | N.D. | N.D. | N.D. | N.D. | N.D. |
| 1c | 0.4 | >18 | N.D. | N.D | 16.4 | 17.3 | 11.7 | N.D | N.D | N.D. | N-D. | N.D. |
| 1d | 0.3 | >18 | N.D. | N.D. | 8.3 | N.D. | N.D. | 10.9 | 8.6 | N.D. | N.D. | N.D. |
| 1) A delamination of the membrane was observed due to repeated depressuring and pressuring during sample collection. With a different test equipment (see example 2 below) the membrane was long term stable. Thus, the effects observed were not caused by the membranes. | | | | | | | | | | | | |

The results in Table 3 show, that different membranes are suitable to reduce the Gardner Color Scale of the CNSL, even in this very simple test set-up, to less than halve of the value of the crude CNSL. Compared to the oNF-2 membranes, made entirely of polydimethylsiloxane, the silicone coated polyimide membrane used in example 1a had advantage in view of the purification efficiency and also of the flow rates. Nevertheless, the tests showed that in principle different membranes can be used in the process of the invention.

Figure 5 shows the results of a GPC analysis of example 1d. It shows, that the membrane separation leads to a reduction of polymers and oligomers in sample 1d compared to the crude CNSL. NMR analysis in addition shows, that the cardol to cardanol ratio was nearly not affected by the membrane separation with a Cardanol to Cardol ratio of 87 : 13 in comparison to a Cardanol : Cardol ratio of 82 : 18 in the feed.

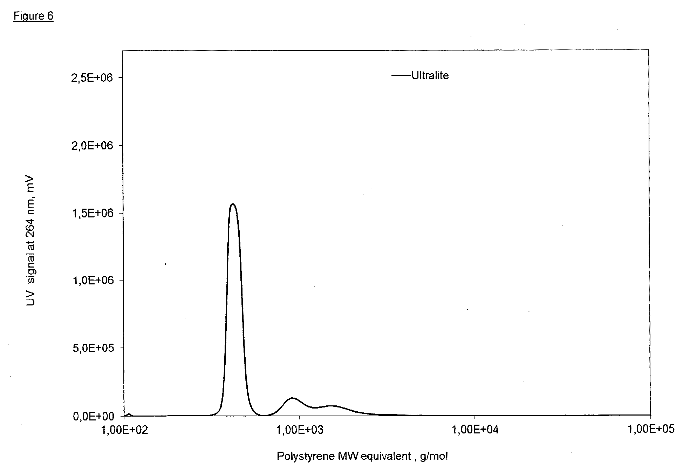

Figure 6 as comparison example shows a GPC analysis of Ultralite 2023, a commercially available purified CNSL. Ultralite 2023 was purified by distillation and shows a very low Gardner Color of 2. The GPC and the NMR analysis confirms, that Ultralite 2023 does not comprise cardol anymore. NC700, another product by Cardolite, shows a Cardanol to Cardol ratio of 92 : 8 with a Gardner Color of 12. Thus, the products obtained by the present invention clearly differ from products obtained by prior art processes.

**Example 2 - CNSL purification in Cross-Flow Cell**

The METcell cross-flow filtration apparatus (Evonik Membrane Extraction Technology Ltd., London, U.K.) consisted of an 800 mL capacity feed vessel and a pumped recirculation loop through two to five cross-flow cells connected in series. The cross-flow system is shown schematically in Figure 3. The mixing in the cross-flow cells was provided by flow from the gear pump: the flow was introduced tangentially to the membrane surface at the outer diameter of the membrane disk and followed a spiral flow pattern to a discharge point at the center of the filtration cell/disk. The nanofiltration membrane disks were conditioned with the experimental solvent at the operating pressure and 30°C until a constant flux was obtained, to ensure that any preservatives/conditioning agents were washed out of the membrane, and maximum compaction of the membrane was obtained.

The test mixture was then permeated across each conditioned membrane disk at the desired operating temperature and pressure. The test were conducted at 45 °C and 50 bar $N_2$ pressure. Samples of the permeate and retentate were collected for analysis every 2 hours.

PuraMem™ S600 (see Table 1) was used as membrane in all examples

The raw materials and solvents used are given in Table 4 below.

Table 4

| Example | Raw material for feed | Solvent used for membrane separation |
|---|---|---|
| 2a | Crude CNSL as in example 1 | No solvent |
| 2b | 50 % by volume crude CNSL and 50 % by volume hexane | hexane |

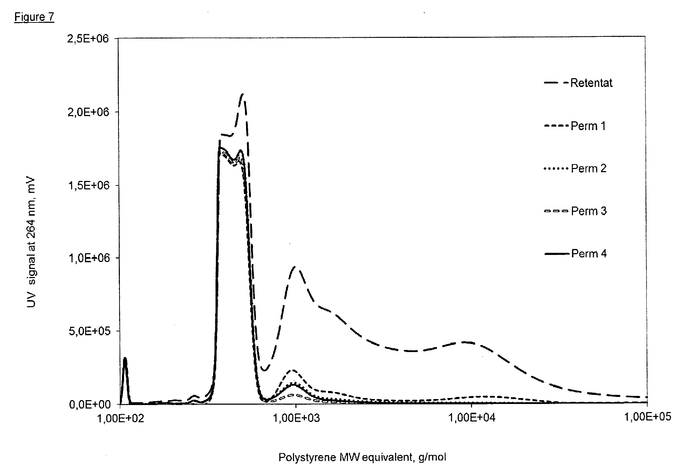

The flow rate in example 2a was in a range of from 1.4 to 3.8 kg/m$^2$h. 4 samples were taken. The Gardner Color Scale of all for samples was in the range of from 2 to 8. The GPC analysis of one retentate sample as well of all 4 permeate samples are shown in Figure 7. It can be seen, that oligomers and polymers are strongly enriched in the permeate while they are strongly depleted in the permeate. The ratio of cardol/cardanol to polymers/oligomers was decreased in the retentate while it was strongly increased in the permeate. Figure 7 shows, that in permeate 4, i.e. after 8h operation, all polymers were filtered out of the permeate and only a very small amount of oligomers remained. It can be expected that continuation of filtration would lead to a nearly quantitative separation of oligomers, too.

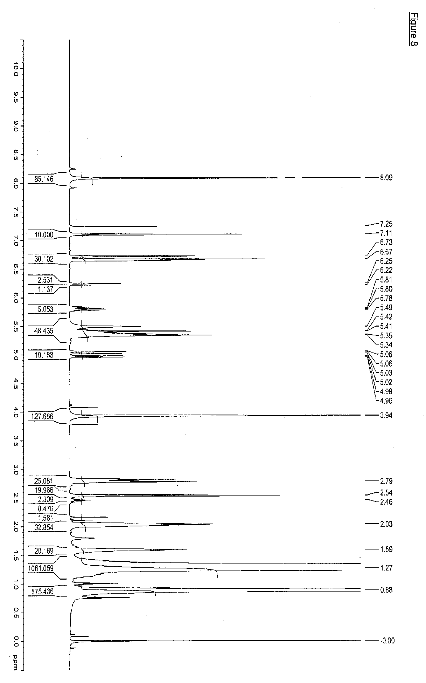

The flow rate in example 2b was in a range from 12.2 kg/m$^2$h to 24.5 kg/m$^2$h 4 samples were taken The Gardner Color Scale of all for samples was in the range of from < 1 to 3. The GPC analysis of one retentate sample as well of all 4 permeate samples are shown in Figure 8. It can be seen, that oligomers and polymers are strongly enriched in the retentate while they are strongly depleted in the permeate. The ratio of cardol/cardanol to polymers/oligomers was decreased in the retentate while it was strongly increased in the permeate. Figure 8 shows, that in permeate 4, i.e. after 8h operation, all polymers and oligomers were filtered out.

Comparison of example 2a and 2b shows, that the use of a diluted crude CNSL leads to a very strong improvement of the flow rates. In addition it shows, that the separation efficiency is strongly increased by using diluted raw materials. In the diluted case, a better purification effect can be achieved at less operating time.

Also storing test were made with 4 permeates samples of examples 2a and 2b at room temperature under air. It showed that the permeate of example 2a tends to re-coloration after 2 days, i.e. is suitable for short-term use while a remarkable re-coloration of the permeate of example 2b could only be observed after 25 days. Thus, the permeate of the diluted process is suitable for long-term use. Figures 7 also shows, that in example 2a a high cardol yield was obtained. Due to the dilution in example 2b the cardol yield could not be determined via GPC analysis. The cardanol: cardol ratio content was, therefore, analyzed by $^1$H-NMR. The ratio was calculated as 89:11 cardanol to cardol.

**Examples 3 and 4 - Storage Test**

For storage tests, crude CNSL was purified as described in example 2. Thereafter two test series were conducted. In a first series (Example 3) purified CNSL diluted with 50wt% heptane with an initial Gardner Color Index of 0.6 and in a second series (Example 4) un-dissolved, i.e. pure purified CNSL with an initial Gardner Color Index of 4.9 was used.

For all storage tests different possible stabilizers were mixed with the CNSL solution respectively the pure CNSL in an amount of 5000 ppm. All samples as well as a blind sample without stabilizer were stored under air at 20°C for 105 days. After 29 days the Gardner color Index, the iodine number and the yellowness index were measured to determine the efficiency of the potential stabilizer. After 105 days the measurements were repeated. The results are summarized in Table 5 and Table 6 below:

**Table 5**

| No. | Tested Substance | in 50 wt% heptane diluted CNSL at 20 °C, 29 days | | | In 50 wt% heptane diluted CNSL at 20 °C, 105 days | | |
|---|---|---|---|---|---|---|---|
| | | Gardner Color Index | Yellowness Index | Iodine Number | Gardner Color Index | Yellowness Index | Iodine Number |
| 3.1 | Thioglycolic acid | 0.60 | 7.90 | 0.80 | 0.5 | 8.4 | 0.7 |
| 3.2 | Triethylphospite | 0.60 | 8.20 | 0.80 | 1.8 | 17.4 | 1.7 |
| 3.3 | Phosphoric acid | 1.30 | 13.40 | 1.40 | 2.7 | 22.7 | 2.4 |
| 3.4 | Oxalic acid | 1.30 | 13.70 | 1.40 | 2.7 | 23.4 | 2.4 |
| 3.5 | Dequest 2066A (Diethylenetriamine penta (methylene phosphonic acid), sodium salt) | 2.10 | 17.60 | 1.90 | 3.2 | 26.1 | 24.8 |
| 3.6 | TNPP (Tris(nonylphenyl) phosphit) | 1.10 | 10.90 | 1.10 | 4.0 | 32.9 | 4.0 |
| 3.7 | Polyacrylic acid | 1.90 | 13.60 | 1.80 | 4.1 | 34.1 | 4.2 |
| 3.8 | Salicylic acid | 1.80 | 14.50 | 1.70 | 4.5 | 39.5 | 5.0 |
| 3.9 | Sodium bisulfite | 3.90 | 26.70 | 3.80 | 5.5 | 56.5 | 7.1 |
| 3.10 | 1- Dodecanthiole | 4.40 | 35.40 | 4.70 | 6.0 | 62.7 | 8.7 |
| 3.11 | Dimethylmaleate | 4.30 | 30.80 | 4.50 | 6.3 | 69.0 | 3.9 |
| 3.12 | Triphenylphosphine | 2.50 | 17.10 | 2.30 | 6.4 | 64.8 | 10.6 |
| 3.13 | DEHA (N,N-Dietehylhydroxylamine) | 4.00 | 29.60 | 4.10 | 6.5 | 75.0 | 11.0 |
| 3.14 | Citrtic acid | 3.80 | 25.30 | 3.50 | 6.5 | 74.0 | 10.8 |
| 3.15 | PTZ (Phenothiazine) | 4.70 | 32.80 | 5.30 | 6.5 | 63.4 | 10.9 |
| 3.16 | Ascorbinic acid | 4.20 | 35.90 | 4.40 | 6.8 | 82.1 | 12.3 |
| 3.17 | Ionol K72 (blend of >72% 2,4-dimethyl-6-tertbutylphenol and other butylated phenols) | 4.30 | 31.10 | 4.60 | 7.0 | 81.5 | 13.4 |
| 3.18 | Ionol 75 (blend of >75% 2,6-di-tert.-butylphenol and other butylated phenols) | 4.50 | 33.80 | 5.00 | 7.0 | 82.6 | 13.6 |
| 3.19 | Na-Sodium thiosulfate | 4.50 | 33.10 | 4.80 | 7.0 | 83.7 | 13.5 |
| 3.20 | MeHQ (4-Methoxyphenol) | 4.70 | 35.70 | 5.20 | 7.0 | 82.4 | 13.7 |
| 3.21 | Ionol 220 (4,4'-M-ethylene-bis-(2,6-di-tert.-butylphenol) | 4.40 | 32.70 | 4.80 | 7.2 | 85.6 | 14.4 |
| 3.22 | EDTA-$Na_2$ (Ethylenediaminetetraacetic acid disodium salt dehydrate) | 4.60 | 34.40 | 5.10 | 7.2 | 85.6 | 14.9 |

(continued)

| No. | Tested Substance | in 50 wt% heptane diluted CNSL at 20 °C, 29 days | | | In 50 wt% heptane diluted CNSL at 20 °C, 105 days | | |
|---|---|---|---|---|---|---|---|
| | | Gardner Color Index | Yellownes s Index | Iodine Number | Gardner Color Index | Yellowness Index | Iodine Number |
| 3.23 | BHT (2,6-Di-tert-butyl-4-methylphenol) | 4.40 | 32.20 | 4.80 | 7.3 | 88.4 | 14.9 |
| 3.24 | Benzoic acid | 4.60 | 40.60 | 5.10 | 7.3 | 92.7 | 15.2 |
| 3.25 | Ionol K65 (blend of >55% 2,4-dimethyl-6-tert.-butylphenol, >15% 2,6-di-tert.-butyl-4-methylphenol and other butylated phenols) | 4.50 | 32.40 | 4.90 | 7.5 | 91.3 | 16.1 |
| 3.26 | styrene | 4.70 | 36.00 | 5.30 | 7.5 | 91.5 | 16.1 |
| REF | **Blind** | **4.60** | **34.40** | **5.00** | **7.6** | **93.5** | **16.5** |
| C1 | TBC (4-tert. Butyl-catechol) | 5.60 | 55.70 | 7.30 | 7.9 | 93.917.8 | - |
| C2 | Glycine | 4.60 | 34.20 | 5.10 | 8.2 | 117.6 | 24.8 |
| C3 | 8-Hydroxy-Quinoline | 7.90 | 95.00 | 17.90 | 10.0 | 145.8 | 62.6 |
| C4 | PDA (N,N'-Di-sec-butyl-p-phenylendiamine) | 11.0 | 151.6 | 87.8 | - | - | - |
| C5 | TMOF (Trimethylortho-formate) | 11.50 | 134.40 | 108.30 | 11.7 | 147.5 | 121.7 |
| C6 | Aq. Formaldehyd - solution | | | | 14.3 | 209.7 | - |
| C7 | Paraformaldehyde | 12.3 | 157.7 | | - - | - | - |
| C8 | Ethylenediamine | 11.90 | 161.10 | | 15.8 | 209.5 | - |
| C9 | HTEMP (4-Hydroxy-TEMPO) | | 302.70 | | 18.7 | 269.0 | - |
| C10 | L-Cysteine | | 267.30 | | - | 247.7 | - |

**Table 6**

| No. | Tested Substance | pure CNSL at 20 °C,. 29 days | | | pure CNSL at 20 °C,. 105 days | | |
|---|---|---|---|---|---|---|---|
| | | Gardner Color Index | Yellowness Index | Iodine Number | Gardner Color Index | Yellowness Index | Iodine Number |
| 4.1 | Thioglycolic acid | 4.90 | 51.10 | 5.60 | 4.9 | 51.9 | - |
| 4.2 | TNPP | 5.60 | 58.00 | 7.20 | 6.5 | 73.4 | 11.1 |
| 4.3 | Phosphoric acid | 5.90 | 67.20 | 8.40 | 6.7 | 81.6 | 12.1 |
| 4.4 | Acrylic acid | 6.70 | 79.00 | 12.10 | 8.0 | 95.5 | 18.7 |
| 4.5 | Oxalic acid | 6.70 | 76.00 | 12.00 | 8.0 | 97.0 | 19.1 |
| 4.6 | Polyacrylic acid | 6.50 | 75.10 | 10.90 | 8.2 | 109.8 | 25.5 |
| 4.7 | Salicylic acid | 6.60 | 77.40 | 11.50 | 8.2 | 106.6 | 25.1 |
| 4.8 | Benzoic acid | 6.90 | 80.20 | 13.10 | 8.4 | 109.8 | 28.8 |
| 4.9 | Triphenylphosphine | 6.50 | 64.80 | 10.70 | 8.7 | 98.8 | 34.6 |

(continued)

| No. | Tested Substance | pure CNSL at 20 °C,. 29 days | | | pure CNSL at 20 °C,. 105 days | | |
|---|---|---|---|---|---|---|---|
| | | Gardner Color Index | Yellowness Index | Iodine Number | Gardner Color Index | Yellowness Index | Iodine Number |
| 4.10 | Dequest 2066A | 7.80 | 91.60 | 17.40 | 8.9 | 127.9 | 39.7 |
| 4.11 | Acetic acid | 8.00 | 98.90 | 19.50 | 8.9 | 128.6 | 39.2 |
| 4.12 | DEHA | 5.60 | 51.70 | 7.30 | 9.0 | 139.4 | 42.0 |
| 4.13 | Triethylphospite | 7.10 | 73.90 | 14.00 | 9.1 | 126.5 | 43.1 |
| 4.14 | 1-Dodecanethiole | 8.10 | 94.90 | 23.60 | 9.7 | 121.3 | 55.0 |
| 4.15 | PTZ | 8.40 | 96.40 | 28.80 | 10.9 | 169.5 | - |
| 4.16 | Marlon AS3 | 9.60 | 124.70 | 53.20 | 11.9 | 157.9 | - |
| 4.17 | Sodium bisulfite, | 8.80 | 124.30 | 37.60 | 12.0 | 230.4 | - |
| 4.18 | TMOF | 14.40 | 171.40 | | 15.8 | 189.6 | - |
| 4.19 | TBC | 8.60 | 120.60 | 33.00 | 17.0 | 215.6 | - |
| 4.20 | Paraformaldehyde | 15.10 | 206.70 | | 17.1 | 255.3 | - |
| 4.21 | PDA | 15.80 | 205.50 | | 17.1 | 256.5 | - |
| 4.23 | Dimethylmaleate | 8.80 | 126.20 | 37.60 | 17.4 | 231.6 | - |
| 4.24 | styreneStyrene | 9.00 | 134.90 | 41.50 | 17.4 | 238.7 | - |
| 4.25 | Ascorbinic acid | 8.70 | 122.10 | 35.00 | 17.6 | 240.6 | - |
| 4.26 | Citric acid | 8.70 | 122.40 | 35.70 | 17.6 | 236.9 | - |
| 4.27 | Ionol K65 | 8.90 | 128.40 | 39.20 | 17.6 | 239.7 | - |
| 4.28 | MeHQ | 8.70 | 124.00 | 36.40 | 17.7 | 239.0 | - |
| 4.29 | BHT | 8.90 | 128.10 | 38.80 | 17.7 | 241.7 | - |
| 4.30 | Ionol 220 | 8.90 | 127.60 | 38.80 | 17.7 | 242.9 | - |
| 4.31 | Ionol K72 | 8.90 | 128.20 | 39.20 | 17.7 | 240.5 | - |
| 4.32 | Ionol 75 | 9.00 | 132.40 | 42.00 | 17.7 | 245.5 | - |
| 4.33 | Sodium thiosulfate | 9.00 | 131.90 | 40.60 | 17.7 | 244.4 | - |
| 4.34 | EDTA-Na$_2$ | 9.00 | 133.40 | 42.00 | 17.7 | 241.2 | - |
| REF | **Blind** | **9.00** | **131.90** | **41.50** | **17.8** | **249.8** | - |
| C11 | 8-Hydroxy-Quinolinequinoline | 15.40 | 182.50 | | 17.9 | 259.8 | - |
| C12 | HTEMPO | | 299.20 | | 17.9 | 273.7 | - |
| C13 | Glycine | 8.90 | 130.20 | 40.10 | 18.4 | 271.6 | - |
| C14 | Ethylenediamine | 14.90 | 176.30 | | 18.8 | 306.7 | - |
| C15 | L-Cysteine | | 315.40 | | - | 252.7 | - |

Table 5 and table 6 show that several acids like thioglycolic acid, phosphoric acid, oxalic acid, salicylic acid, polyacrylic acid, benzoic acid and Dequest 2066a stabilize diluted CNSL as well as pure CNSL for 29 as well as for 105 days.

Other acids like acrylic acid, acetic acid and docecylbenze sulfonic acid can be used for stabilization of pure CNSL for short term (29 days) as well as for long term (105 days). For CNSL diluted in inpolar and/or non-protic solvents these acids are less effective than the acids discussed in the paragraph before due to limited deprotonation.

Acids like ascorbic acid and citric acid also showed some stabilization potential for diluted as well as for pure CNSL even though their stabilization potentials seems somewhat lower that that of the acids discussed before.

Oxygen scavengers like triethylphosphite, TNPP, trihenylphosphine, 1-Dodecanthiol, PTZ, sodium bisulfite and diethylhydroxyl amine proved to be suitable for stabilizing crude and diluted CNSL.

The best overall performance showed thioglycolic acid, which combines a carboxylic acid functionality and an oxygen scavenging functionality in one molecule.

Several potential stabilizers and radical polymerization inhibitors were tested, too, but did not show any stabilization effect. Some tested substance even increases the coloration rate instead of the desired decrease.

Table 5 and 6 prove, that compositions according to the present invention can be storage stabilized by addition of suitable agents. Acids, their salts and oxygen scavenging compounds are preferred and showed the best results. Most preferred would be a combined use of acids and oxygen scavengers, either in different molecules or both combined in one molecule.

**Claims**

1. A process for separating valuable components from impurities in a CNSL, i.e. a cashew nut shell liquid, to obtain a purified CNSL, wherein the process comprises the following steps:

   (i) providing a crude CNSL or a solution comprising a crude CNSL and at least one miscible organic solvent by isolation of the crude CNSL from the honeycomb of the shell of a cashew nut and optionally mixing the isolated CNSL with at least one solvent,
   (ii) optionally pre-purifying and/or refining the crude CNSL or the solution comprising crude CNSL,
   (iii) providing a selectively permeable nanofiltration membrane having a first surface and a second surface;
   (iv) separating components of the crude CNSL or the solution comprising crude CNSL from step (i) or (ii) by transferring one or more components of the crude CNSL or of the crude CNSL solution from the first surface to the second surface across the membrane through contacting the crude CNSL or the solution comprising crude CNSL from step (i) or (ii) with the first surface, so that the components of the crude CNSL or the solution comprising crude CNSL from step (i) or (ii) in contact with the first surface form a retentate and the components of the crude CNSL or the solution comprising crude CNSL from step (i) or (ii) contacting the second surface form a permeate,

   the process is further **characterized in that**
   the pressure at the first membrane surface is greater than the pressure at the second membrane surface, and
   the crude CNSL or the solution comprising crude CNSL used in step (iv) comprises as valuable compounds cardol and/or cardanol and optionally one or two compound(s) selected from the group consisting of 2-methylcardol and anacardic acid; it further comprises at least one, preferably two or more impurities selected from the group consisting of polymers, oligomers, and residues from cashew nut shells and
   at least one, preferably two, more preferred three, valuable compound(s) is/are enriched and at least one, preferably two, more preferred three, impuritie(s) is/are depleted in the permeate compared to the crude CNSL.

2. The process according to claim 1,
   **characterized in that** the crude CNSL is obtained in step (i) from a natural source, by a process comprising exposure of CNSL to temperatures above 150°C, preferably by contacting the natural source with hot CNSL or by roasting it at the desired temperature.

3. The process according to claim 1,
   **characterized in that** the crude CNSL is obtained in step (i) from a natural source, by a process avoiding exposure of the CNSL to temperatures above 150°C, preferably by pressing out a natural source or extraction of it with organic solvents.

4. The process according to any one of claims 1 to 3, further comprising recovering any solvent content from the permeate and/or retentate solutions.

5. The process according to any one of claims 1 to 4,
   **characterized in that** the purified CNSL and/or purified CNSL solution after step (iv) is subjected to at least one additional processing step, preferably a refining step and/or a purification step.

**6.** The process according to claim 5,
**characterized in that** the permeate and/or the retentate obtained from step (iv) is subjected to at least one additional contact with a selectively permeable membrane, preferably a nanofiltration membrane.

**7.** The process according to any one of claims 1 to 6
**characterized in that** the at least one impurity comprises polymers and/or oligomers formed from at least one valuable component of the crude CNSL.

**8.** The process according to any one of the preceding claims,
**characterized in that** the organic solvent used to prepare the mixture of CNSL and organic solvent is chosen from aromatic hydrocarbons, aliphatic hydrocarbons, ketones, glycols, chlorinated solvents, esters, ethers, amines, nitriles, aldehydes, phenols, amides, carboxylic acids, alcohols, furans, lactones, and dipolar aprotic solvents, and mixtures thereof and with water
and/or
from toluene, xylene, benzene, styrene, anisole, chlorobenzene, dichlorobenzene, chloroform, dichloromethane, dichloroethane, methyl acetate, ethyl acetate, isopropyl acetate ,butyl acetate, methyl ether ketone (MEK), methyl iso butyl ketone (MIBK), acetone, ethylene glycols, ethanol, methanol, isopropanol, propanol, butanol, pentane, hexane, heptane, petrolether, cyclohexane, dimethoxyethane, methyl tert butyl ether (MTBE), diethyl ether, adiponitrile, N,N dimethylformamide, dimethylsulfoxide, N,N dimethylacetamide, dioxane, nitromethane, nitrobenzene, pyridine, carbon disulfide, tetrahydrofuran, methyl-tetrahydrofuran, N-methyl pyrrolidone, N-ethyl pyrrolidone, acetonitrile, and mixtures thereof and with water;
preferably from methanol, ethanol, isopropanol, ethyl acetate, acetone, hexane, heptane, ethylene glycols, and mixtures thereof and with water.

**9.** The process according to any one of the preceding claims,
**characterized in that** at least one selective nanofiltration membrane comprises a material chosen from
polyethylene, polypropylene, polytetrafluoroethylene (PTFE), polyvinylidene difluoride (PVDF), polysulfone, polyethersulfone, polyacrylonitrile, polyamide, polyimide, polyamideimide, polyetherimide, cellulose acetate, polyaniline, polypyrrole, polyetheretherketone (PEEK), polybenzimidazole, and mixtures thereof,
or from
a composite material comprising a support and a thin, selectively permeable layer, preferably a thin, selectively permeable layer comprising a material chosen from modified polysiloxane based elastomers including polydimethylsiloxane (PDMS) based elastomers, ethylene-propylene diene (EPDM) based elastomers, polynorbornene based elastomers, polyoctenamer based elastomers, polyurethane based elastomers, butadiene and nitrile butadiene rubber based elastomers, natural rubber, butyl rubber based elastomers, polychloroprene (Neoprene) based elastomers, epichlorohydrin elastomers, polyacrylate elastomers, polyethylene, polypropylene, polytetrafluoroethylene (PTFE), polyvinylidene difluoride (PVDF) based elastomers, polyetherblock amides (PEBAX), polyurethane elastomers, crosslinked polyether, polyamide, polyaniline, polypyrrole, and mixtures thereof, particular preferred a thin, selectively permeable layer comprising a polysiloxane based elastomer
or from
a material comprising an inorganic material chosen from silicon carbide, silicon oxide, zirconium oxide, titanium oxide, and zeolites
or from
a polymer membrane with dispersed organic or inorganic matrices in the form of powdered solids present in amounts up to about 20 wt% of the polymer membrane.

**10.** The process according to claim 9,
**characterized in that** at least one selective nanofiltration membrane
comprises a polyimide, preferably a polyimide subject to post-formation crosslinking and impregnation, particular preferred having a molecular weight cut-off ranging from about 150 g/mol to about 1,500 g/mol, more preferred from about 200 g/mol to about 800 g/mol and most preferred 200 g/mol to less than or equal to 600 g/mol,
and/or
comprises silicone-coated organic solvent nanofiltration membranes, preferably on the basis of polyimide nanofiltration membranes, particular preferred having a molecular weight cut-off ranging from about 150 g/mol to about 1,500 g/mol, more preferred from about 200 g/mol to about 800 g/mol and most preferred 200 g/mol to less than or equal to 600 g/mol.

**11.** The process according to any one of the preceding claims,

**characterized in that**
the process, in particular the nanofiltration process, is performed at a temperature ranging from about 0 to 80°C, preferred 5 to 70°C, more preferred 10 to 60°C and most preferred 20 to 50 °C and/or
that the trans-membrane pressure in step (iv) is in a range of from about 1 to 100 bar, preferably 5 to 75 bar, particular preferred 10 to 60 bar and/or
that the CNSL solution after steps (i) or (ii) contains crude CNSL in an amount ranging of from about 1% v/v to about 99% v/v, preferably 50 to 95% v/v, more preferably 60 to 90% v/v, most preferably 65 to 85 % v/v.

12. The process according to any one of the preceding claims,
   **characterized in that**
   more stabilizing agents(s), preferably selected from the group consisting of acids or their salts, oxygen scavengers, phenothiazines, phenolic stabilizers and mixtures thereof, more preferred selected from acids or their salts, oxygen scavengers and mixtures thereof, are added in one or more portions before, during or after any one of steps (i) to (iv), preferably after step (iv).

13. Product mixture,
   **characterized in that** all valuable components are of natural origin and that it comprises a mixture of:

   a) Anacadic acid: 35 - 80 % by weight of the mixture; preferably 40 - 75 % by weight of the mixture, particular preferred 45 - 70 % by weight of the mixture
   b) Cardol 5 - 40 % by weight of the mixture; preferably 10 - 35 % by weight of the mixture, particular preferred 10 - 30 % by weight of the mixture and most preferred 10 - 25 % by weight of the mixture
   c) Cardanol 5 - 30 % by weight of the mixture, preferably 5 - 25 % by weight of the mixture, particular preferred 10 - 25 % by weight of the mixture
   d) 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture
   e) Polymers and oligomers 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture,

   wherein the amounts of components a) to e) are selected in a manner that the sum of components a) to e) in the total mixture of a) to e) calculates to 100 % by weight.

14. Product mixture,
   **characterized in that** all valuable components are of natural origin and that it comprises a mixture of:

   A. Anacadic acid: 0 - 5 % by weight of the mixture, preferably 0,1 - 4 % by weight of the mixture, particular preferred 0,5 - 3 % by weight of the mixture, even more preferred 1 - 2 % by weight of the mixture
   B. Cardol 2 - 35 % by weight of the mixture; preferably 3 - 30 % by weight of the mixture, particular preferred 4 - 25 % by weight of the mixture, most preferred 5 - 20 % by weight of the mixture
   C. Cardanol 55 - 95 % by weight of the mixture, preferably 60 - 90 % by weight of the mixture, particular preferred 65 - 85 % by weight of the mixture
   D. 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture
   E. Polymers and oligomers 2-Methylcardol 0 - 5 % by weight of the mixture, preferably 0,1 - 4 % by weight of the mixture, particular preferred 0,5 - 3 % by weight of the mixture, even more preferred 1 - 2 % by weight of the mixture

   wherein the amounts of components A) to E) are selected in a manner that the sum of components A) to E) in the total mixture of A) to E) calculates to 100 % by weight.

15. Product mixture according to claim 13 or 14
   **characterized in that**
   it further comprises one or more solvent(s) in an amount in sum of from1 to 95 % by weight, preferably of from 10 to 90 % by weight, more preferred of from 20 to 80 % by weight and most preferred of from 30 to 70 % by weight, "% by weight" means "by weight of the total composition",
   and/or
   it further comprises one or more stabilizing agent(s) selected from the group consisting acids or their salts, oxygen scavengers, phenothiazines, phenolic stabilizers and mixtures thereof, preferably selected from acids or their salts, oxygen scavengers and mixtures thereof, in an amount in sum of from 20 to 0.0001 % by weight, preferably of from 10 to 0.0005 % by weight, more preferred of from 1 to 0.001 % by weight and most preferred of from 0.1 to 0.01 % by weight, "% by weight" means "by weight of the total composition".

Figure 1 (Part 1 of 2)

(I) Cardol

(II) Cardanol

(III) Anarcardic Acid

(IV) 2-Methyl-cardol

wherein R = $C_{15}H_{31-n}$ with n = 0, 2, 4, 6, preferably as shown in Formulas V to VIII below

Figure 1 (Part 2 of 2)

$n = 0$    (V)

$n = 2$    (VI)

$n = 4$    (VII)

$n = 6$    (VIII)

Figure 2

High pressure
regulator

High pressure
tube or hose

Flexible hose

Inert gas
cyclinder

METcell

METcell gas
control unit

Balance

Magnetic stirrer

Figure 3

Pressure

N₂

HPLC pump

P

Feed vessel

Re-circulation
pump

T

Retentate
Sampling
port

Heater

Permeate sampling ports

Permeate
collection
vessel

Figure 4

Solvent Reservoir

Batch Feed Tank

10

11

12

13

14

15

16

17

18

19

20

Membrane

Solvent to fed system at same rate permeate flows through membrane

→ Diafiltration

Figure 5

Figure 6

Figure 7

Polystyrene MW equivalent, g/mol

Figure 8

Europäisches
Patentamt

European
Patent Office

Office européen
des brevets

# EUROPEAN SEARCH REPORT

Application Number

EP 16 17 2170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | P.H. GEDAM ET AL: "Cashew nut shell liquid: Extraction, chemistry and applications", PROGRESS IN ORGANIC COATINGS, vol. 14, no. 2, 1 October 1986 (1986-10-01), pages 115-157, XP055111008, ISSN: 0300-9440, DOI: 10.1016/0033-0655(86)80009-7 | 1-6,8-14 | INV. B01D61/02 C07C37/82 C07C37/88 C07C39/06 C07C39/08 C07C39/19 C07C51/47 C07C51/50 |
| Y | * page 119; table 2; compound II * | 15 | C07C65/05 C07C65/19 |
| Y,D | EP 2 578 670 A1 (IDEMITSU KOSAN CO [JP]) 10 April 2013 (2013-04-10) * claims; examples * | 15 | C07C27/26 |
| A | GB 2 254 323 A (UNIV BRUNEL [GB]) 7 October 1992 (1992-10-07) * the whole document * | 1-15 | |
| A | JP H05 979 A (TOHOKU KAKO KK) 8 January 1993 (1993-01-08) * the whole document * | 1-15 | TECHNICAL FIELDS SEARCHED (IPC) |
| A | SEE TOH ET AL: "In search of a standard method for the characterisation of organic solvent nanofiltration membranes", JOURNAL OF MEMBRANE SCIENCE, ELSEVIER BV, NL, vol. 291, no. 1-2, 1 March 2007 (2007-03-01), pages 120-125, XP005908878, ISSN: 0376-7388, DOI: 10.1016/J.MEMSCI.2006.12.053 * the whole document * | 1 | B01D C07C |

The present search report has been drawn up for all claims

| Place of search | Date of completion of the search | Examiner |
|---|---|---|
| The Hague | 15 November 2016 | Kardinal, Siegmar |

EPO FORM 1503 03.82 (P04C01)

## ANNEX TO THE EUROPEAN SEARCH REPORT
## ON EUROPEAN PATENT APPLICATION NO.

EP 16 17 2170

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2016

| Patent document cited in search report | | Publication date | Patent family member(s) | Publication date |
|---|---|---|---|---|
| EP 2578670 | A1 | 10-04-2013 | AR       083151 A1 | 06-02-2013 |
| | | | AU   2011259858 A1 | 10-01-2013 |
| | | | BR 112012030643 A2 | 16-08-2016 |
| | | | CA      2801286 A1 | 08-12-2011 |
| | | | CN    102933696 A | 13-02-2013 |
| | | | EP      2578670 A1 | 10-04-2013 |
| | | | JP      5802662 B2 | 28-10-2015 |
| | | | KR  20130083390 A | 22-07-2013 |
| | | | NZ       604839 A | 25-07-2014 |
| | | | US   2013071530 A1 | 21-03-2013 |
| | | | WO   2011152532 A1 | 08-12-2011 |
| GB 2254323 | A | 07-10-1992 | NONE | |
| JP H05979 | A | 08-01-1993 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 152925 A
- WO 2006108545 A
- WO 2006108546 A
- GB 2066820 A
- GB 2437519 A

- DE 102009047351
- WO 2012010889 A
- US 6585802 B
- US 6755900 B
- EP 2578670 A

### Non-patent literature cited in the description

- **A. R. R.MENON.** *J. Sci. Ind. Res,* 1985, vol. 44, 324
- **P. H. GEDAM.** *Prog. Org. Coat,* 1986, vol. 14, 115
- **J. H. P. TYMAN.** *Chem. Soc. Rev,* 1973, vol. 8, 499-537
- **J. H. P. TYMAN.** *J. Am. Chem. Soc.,* 1978, vol. 55, 663
- **P. A. MAHANWAR.** *Indian J. Technol,* 1996, vol. 3, 191-193
- *Journal of Membrane Science,* 2007, vol. 291 (1-2), 120-125

- **RODRIGUES F.H.A.** *Polimeros,* 2011, vol. 21 (2), 156-160
- **MAZZETTO, G.** *Quim. Nova,* 2009, vol. 32, 732
- **KUMAR, P. P.** *J. Agric. Food Chem.,* 2002, vol. 50, 4705
- **IKEDA, R.** *Polymer,* 2002, vol. 43, 3475
- **LUBI M.C.** *Des. Mon. Pol.,* 2000, vol. 3 (2), 123
- **SOROKO et al.** *Journal of Membrane Science,* 2011, vol. 381 (1-2), 152-162